# EUROPEAN PATENT APPLICATION

(11) **EP 4 198 058 A1**
(43) Date of publication of application: **21.06.2023**
(21) Application number: 21856268.4
(22) Date of filing: 12.08.2021
(51) Int. Cl.: C07K 16/32, C12N 15/62, A61K 38/00, A61P 35/00

(54) **ANTIBODY TARGETING INTRACELLULAR TUMOR-INDUCING PROTEIN, OR FUSION PROTEIN OF SINGLE STRAND VARIABLE FRAGMENT THEREOF AND CANCER-CELL-PENETRATING PEPTIDE, AND USE THEREOF**

(30) Priority: 13.08.2020 KR 20200101760
(71) Applicant: Nibec Co., Ltd., Chungcheongbuk-do 27816 (KR)
(72) Inventor: CHUNG, Chong-Pyoung, Seoul 05618 (KR); PARK, Yoon Jeong, Seoul 08291 (KR); LEE, Jue-Yeon, Gwacheon-si Gyeonggi-do 13831 (KR); KIM, Deog Il, Gwangmyeong-si Gyeonggi-do 14247 (KR); JEONG, Eui Kyun, Seoul 01351 (KR)
(74) Representative: Guardian IP Consulting I/S
(86) International application number: PCT/KR2021/010749
(87) International publication number: WO 2022/035262

(57) **Abstract**

The present invention relates to: an antibody targeting a mutation of KRAS, which is an intracellular tumor-inducing protein, or a fusion protein in which a cancer-cell-penetrating peptide is connected, by means of a gene expression method or a chemical bonding method, to a single strand variable fragment of the antibody; and a tumor treatment use thereof. The fusion protein thus produced has the benefit of maximizing the anti-tumor or anti-cancer effect of the antibody targeting an intracellular tumor-inducing protein or a tumor-inducing mutant protein, or the single strand variable fragment of the antibody, by effectively invading a tumor cell.

## Description

### Technical Field

The present invention relates to a fusion protein comprising an intracellular oncogenic protein-targeting antibody or a single-chain variable fragment thereof; and a cancer cell-penetrating peptide and the use thereof, and more particularly, to a fusion protein wherein a cancer cell-penetrating peptide is linked to an antibody or a single-chain variable fragment thereof, which targets a mutation in the intracellular oncogenic protein KRAS, by a gene expression or chemical conjugation method, and the use thereof for treating tumors.

### Background Art

Tumors are caused by genetic mutations in normal cells due to activation of oncogenic pathways or inhibition of tumor suppressive pathways (1). Among various oncogenic factors, the RAS mutant protein is the most widely known oncogenic factor, and appears in about 30% of all cancer patients. The RAS mutant protein binds to GTP and remains activated, causing proliferation and growth of tumor cells. RAS may be divided into three categories: HRAS, NRAS, and KRAS. KRAS mutations are mainly found in non-small-cell-lung carcinoma, colorectal carcinoma, and pancreatic carcinoma, HRAS mutations are found in bladder carcinoma, kidney carcinoma, and thyroid carcinoma, and NRAS mutations are mainly found in melanoma, hepatocellular carcinoma, and hematologic malignancies (2). KRAS gene mutations account for 86% of cancers caused by RAS mutations, and are known to be found in more than 98% of pancreatic cancer, more than 53% of rectal cancer, and more than 30% of lung adenocarcinoma (3) .

KRAS mutations are known to be deeply involved in the mechanism of resistance to anti-EGFR therapeutics such as Gefitinib, Erlotinib, and Cetuximab, which target epithelial growth factor receptor (EGFR). Even when EGFR inhibitory anticancer drugs are used, they have no effect at all in a state in which KRAS, which is downstream of EGFR, remains activated due to a mutation therein. In fact, administration of an anti-EGFR monoclonal therapeutic antibody in colorectal cancer is possible after determining that the patient does not have a KRAS mutation.

Antibody drugs have been widely studied as a means of anticancer treatment. For example, Trastuzumab is a monoclonal antibody against Her2 protein and is used as a treatment for breast cancer (4), and Rituximab is a monoclonal antibody against CD20 and is used as a treatment for B cell malignant lymphoma (5). Antibodies have an excellent inhibitory effect on the target, but because they cannot pass through the cell membrane due to high molecular weight thereof, it is difficult to obtain the inhibitory effect of the antibody when the target of the antibody is inside the cell.

Prostate cancer is the third most common male cancer in the world, and in the United States, it is the most common male cancer and has the second highest cancer-specific mortality after lung cancer (7). Local prostate cancer can be cured by surgery or radiation therapy, but chemical castration is mainly used as a standard treatment for advanced or metastatic prostate cancer. After it was proved that prostate cancer is testosterone-dependent cancer, artificial castration was established as the primary treatment for advanced or metastatic prostate cancer, and castration treatment for various types of prostate cancer has been used in clinical practice (8). In metastatic prostate cancer, castration shows effects such as disease progression and alleviation of accompanying symptoms, but it is known that only 75% of patients consistently respond to treatment when castration is performed for more than 18 months. Castration shows a relatively good response at the beginning, but over time, prostate cancer cells do not undergo apoptosis due to androgen blockade and are converted into castration-resistant prostate cancer (CRPC) cells which are no longer responsive to hormone treatment (9).

CRPC is defined as a condition in which the testes produce no testosterone so that testosterone in blood is reduced, and even after administration of all drugs that act through androgen receptors is stopped, there is no decrease in prostate specific antigen (PSA), and a significant increase in PSA or radiological progression appears. If CRPC is left untreated, the average survival period is less than 12 months, and following various treatments, the average survival period of metastatic prostate cancer patients is less than 3 years, and in the case of locally invasive disease, the average survival period is only 4.5 years (10).

It is known that androgen receptor (AR) in CRPC is reactivated by various mechanisms such as AR overexpression, mutation, hypersensitivity, and androgen synthesis in the tumor (11).

Abiraterone, an FDA-approved CRPC treatment, is an inhibitor of androgen synthesis, and enzalutamide (MDV3100), a second-generation androgen receptor antagonist, completely inhibits androgen binding to androgen receptors, thus keeping androgen receptors from moving into the nucleus and binding to target genes. Since most prostate cancers that recur after abiraterone or enzalutamide treatment are PSA-positive and androgen receptor is reactivated, new androgen receptor inhibitory therapies are needed for CRPC patients resistant to abiraterone or enzalutamide (12). Androgen receptor, a member of the steroid receptor superfamily, is a ligand-dependent transcription factor that regulates the expression of genes affected by androgens. In order for a transcription factor to access the target gene, the transcription factor must enter the nucleus, and thus if the transcription factor can be maintained only in the cytoplasm, the transcription function can be blocked. Accordingly, the most important step is to control the translocation of androgen receptors into the nucleus. Androgen-sensitive receptors remain in the cytoplasm in the absence of androgens, and move into the nucleus in the presence of androgens and activate target genes. However, in CRPC cells, androgen receptors continue to activate the target genes because they remain in the nucleus even in the absence of androgens (13). Accordingly, if any substance can inhibit androgen receptor translocation into the nucleus, it can be used as an effective treatment for CRPC tumors.

Oncogenic proteins such as KRAS and androgen receptors are known to play an important role in the development of human cancer, but antibody therapeutics targeting these oncogenic proteins have not yet been used clinically. The reason is that the antibody against the RAS and androgen receptor distributed in the cell cannot pass through the cell membrane because of its high molecular weight. It is known that a single-chain variable fragment (scFv) smaller than an antibody has a smaller molecular weight than the antibody and does not have an Fc region that causes immune rejection, and thus it can be an alternative to a general antibody. However, since scFv itself has a molecular weight of 25 kDa or more, it is difficult for scFv to freely pass through the cell membrane, unlike low-molecular-weight drugs, and thus it is difficult to obtain a large inhibitory effect as expected.

Accordingly, the present inventors have made extensive efforts to solve the above-described problems occurring in the prior art, and as a result, have attempted to fuse an antibody against an intracellular oncogenic protein or a single-chain variable fragment thereof with a cancer cell penetrating functional peptide so as to be capable of penetrating cancer tissue. As the fusion method, a gene expression method or a chemical conjugation method was used, and the present inventors have found through *in vitro* and animal experiments that the cell-penetrating fusion protein produced by this method can effectively inhibit the proliferation and growth of cancer cells in which oncogenic proteins are highly expressed, thereby completing the present invention.

### Prior Art

### Non-Patent Literature

(Non-Patent Document 1) Zhang J, Chen YH, Lu Q. Pro-oncogenic and anti-oncogenic pathways: opportunities and challenges of cancer therapy. Future Oncol. 2010 Apr;6(4):587-603.
(Non-Patent Document 2) Cox AD, Fesik SW, Kimmelman AC, Luo J, Der CJ. Drugging the undruggable RAS: mission possible? Nat Rev Drug Discov 2014; 13: 828 - 51.
(Non-Patent Document 3) RAS oncogenes: weaving a tumorigenic web. Pylayeva-Gupta Y, Grabocka E, Bar-Sagi D. Nat Rev Cancer. 2011 Oct 13; 11(11) :761-74.
(Non-Patent Document 4) Trastuzumab (herceptin) for the medical treatment of breast cancer. Bayoudh L, Afrit M, Daldoul O, Zarrad M, Boussen H. Tunis Med. 2012 Jan; 90(1) :6-12.
(Non-Patent Document 5) Evolution of anti-CD20 monoclonal antibody therapeutics in oncology. Oflazoglu E, Audoly LP. MAbs. 2010 Jan-Feb; 2(1) :14-9.
(Non-Patent Document 6) Demarest SJ, Glaser SM. Antibody therapeutics, antibody engineering, and the merits of protein stability. Curr Opin Drug Discov Devel. 2008;11(5):675-687.
(Non-Patent Document 7) Jemal A, Clegg LX, Ward E, Ries LA, Wu X, Jamison PM, et al. Annual report to the nation on the status of cancer, 1975-2001, with a special feature regarding survival. Cancer 2004;101:3-27.
(Non-Patent Document 8) Yagoda A, Petrylak D. Cytotoxic chemotherapy for advanced hormone-resistant prostate cancer. Cancer 1993;71:1098.
(Non-Patent Document 9) Rini BI, Small EJ. Hormone-refractory prostate cancer. Curr Treat Opt Oncol 2002;3:437-46.
(Non-Patent Document 10) Sella A, Yarom N, Zisman A, Kovel S. Paclitaxel, estramustine and carboplatin combination chemotherapy after initial docetaxel-based chemotherapy in castration resistant prostate cancer. Oncology 2009;76:442.
(Non-Patent Document 11) Chen CD, Welsbie DS, Tran C, Baek SH, Chen R, Vessella R, et al. Molecular determinants of resistance to antiandrogen therapy. Nat Med 2004;10:33-9.
(Non-Patent Document 12) Boudadi K, Antonarakis ES. Resistance to novel antiandrogen therapies in metastatic castration-resistant prostate cancer. Clin Med Insights Oncol 2016;10:1-9.
(Non-Patent Document 13) Zhang L, Johnson M, Le KH, Sato M, Ilagan R, Iyer M, et al. Interrogating androgen receptor function in recurrent prostate cancer. Cancer Res 2003;63:4552-60.
(Non-Patent Document 14) Lars Kober, Christoph Zehe, Juergen Bode, Biotechnology and Bioengineering, Vol. 110, No. 4, April, 2013, Optimized signal peptides for the development of high expressing CHO cell lines.

### Summary of the Invention

An object of the present invention is to provide a fusion protein having a significantly improved tumor treatment effect.

Another object of the present invention is to provide a pharmaceutical composition for treating a tumor comprising the fusion protein as an active ingredient.

Still another object of the present invention is to provide a method for preventing or treating a tumor comprising a step of administering the fusion protein.

Yet another object of the present invention is to provide the use of the fusion protein for preventing or treating a tumor.

Still yet another object of the present invention is to provide the use of the fusion protein in the manufacture of a medicament for treating a tumor.

To achieve the above objects, the present invention provides a fusion protein in which (i) an antibody or a single-chain variable fragment thereof, which targets an intracellular oncogenic protein or oncogenic mutant protein, is linked with (ii) a cancer cell-penetrating peptide.

The present invention also provides a nucleic acid encoding the fusion protein.

The present invention also provides a recombinant vector into which the nucleic acid has been introduced.

The present invention also provides a recombinant cell into which the recombinant vector has been introduced.

The present invention also provides a method for producing the fusion protein comprising steps of:
(a) expressing the fusion protein by culturing the recombinant cell; and
(b) recovering the expressed fusion protein.

The present invention also provides a pharmaceutical composition for treating a tumor comprising the fusion protein as an active ingredient.

The present invention also provides a method for preventing or treating a tumor comprising administering the fusion protein.

The present invention also provides the use of the fusion protein for preventing or treating a tumor.

The present invention also provides the use of the fusion protein in the manufacture of a medicament for treating a tumor.

### Brief Description of Drawings

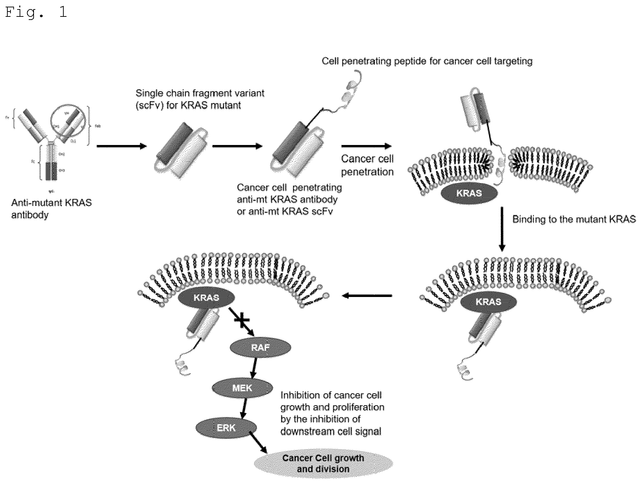

FIG. 1 is a schematic view showing the outline of the present invention.

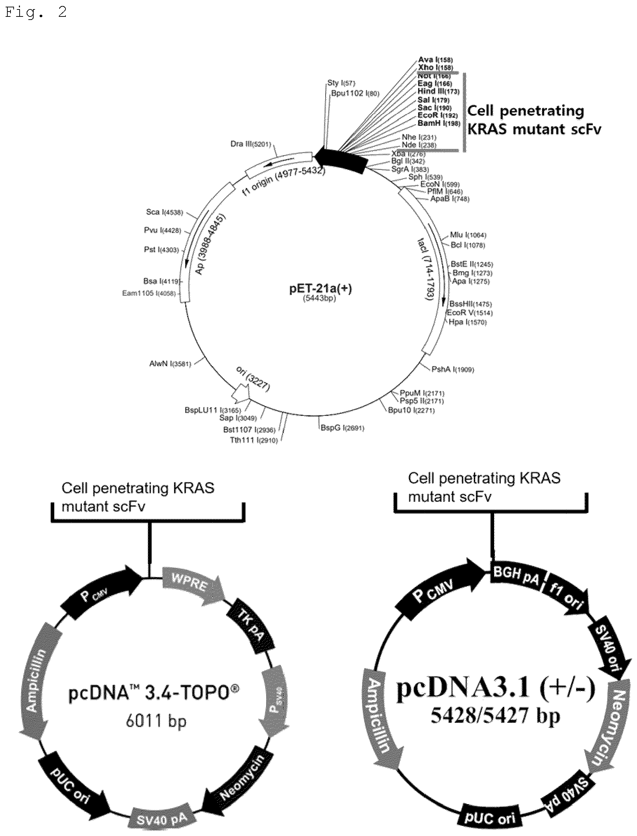

FIG. 2 shows expression vectors, each comprising a cancer cell-penetrating KRAS mutant antibody or scFv.
FIG. 3 shows the results of SDS-PAGE and Western blot analysis of cancer cell-penetrating KRAS mutant antibodies and scFvs after expression and purification.
FIG. 4 shows the results of SDS-PAGE and Western blot analysis of cancer cell-penetrating KRAS mutant antibodies and scFvs produced by a chemical conjugation method.
FIG. 5 shows the results of evaluating the cancer cell-specific killing effects of cancer cell-penetrating KRAS mutant antibodies and scFvs.
FIG. 6 shows the results of Western blot analysis performed to evaluate the active KRAS (mutant KRAS) expression inhibitory activities of SEQ ID NOs: 20 to 23, each linked by a GGGGS linker.
FIG. 7 shows the results of Western blot analysis performed to evaluate the active KRAS (mutant KRAS) expression inhibitory activities of SEQ ID NOs: 90 to 93, each linked by a GGGGSGGGGSGGGGS linker.
FIG. 8 shows the results of analyzing the ability of SEQ ID NOs: 20 to 23 to penetrate cancer cells.
FIG. 9 shows the results of evaluating the distribution of SEQ ID NOs: 2 and 23 in cancer tissue in a tumor xenograft animal model.
FIG. 10 shows the fluorescence intensity of cancer tissue in a tumor xenograft animal model.
FIG. 11 shows the tumor inhibitory effects of SEQ ID NOs: 2 and 23.
FIG. 12 shows changes in volumes of tumors formed by H358 and images of cancer tissue after euthanasia.
FIG. 13 shows the results of Western blot analysis performed to evaluate the active KRAS (mutant KRAS) expression inhibitory activities of SEQ ID NOs: 80 to 83 produced by chemical conjugation.
FIG. 14 shows the results of evaluating the cancer cell-specific killing effects of SEQ ID NOs: 80 to 83 produced by chemical conjugation.
FIG. 15 shows the results of evaluating the cancer cell penetration abilities of SEQ ID NOs: 80 to 83 produced by chemical conjugation.
FIG. 16 shows the results of evaluating the distribution to cancer tissue in a tumor xenograft animal model.
FIG. 17 shows the fluorescence intensity of cancer tissue in a tumor xenograft animal model.
FIG. 18 shows the tumor inhibitory effects of SEQ ID NOs: 3 and 83.
FIG. 19 shows changes in volumes of tumors formed by H358 and images of cancer tissue after euthanasia.

### Detailed Description and Preferred Embodiments of the Invention

Unless otherwise defined, all technical and scientific terms used in the present specification have the same meanings as commonly understood by those skilled in the art to which the present invention pertains. In general, the nomenclature used in the present specification and the experimental methods described below are well known and commonly used in the art.

The present inventors paid attention to the fact that, even if an antibody or a single-chain variable fragment thereof, which targets an intracellular oncogenic protein or oncogenic mutant protein, reaches cancer cells, it cannot sufficiently act on the cancer cells. Accordingly, the present inventors have produced a fusion protein wherein the antibody or single-chain variable fragment thereof is linked with a cancer cell-penetrating peptide. To produce the fusion protein, a linker was introduced to the N-terminus or C-terminus of an antibody or a single-chain variable fragment thereof, which targets an intracellular oncogenic protein or oncogenic mutant protein, and a cancer cell-penetrating peptide was linked thereto. This fusion protein was produced by any one of two different methods: a gene expression method and a chemical conjugation method. In a cancer cell killing experiment conducted using the fusion protein produced as described above, it was confirmed that the fusion protein comprising the cancer cell-penetrating peptide exhibited a significantly better cancer cell-specific effect than the antibody or single-chain variable fragment thereof alone which targets the intracellular oncogenic protein or oncogenic mutant protein.

Therefore, in one aspect, the present invention is directed to a fusion protein in which (i) an antibody or a single-chain variable fragment thereof, which targets an intracellular oncogenic protein or oncogenic mutant protein, is linked with (ii) a cancer cell-penetrating peptide.

In the present invention, the intracellular oncogenic protein or oncogenic mutant protein may be KRAS or androgen receptor, but is not limited thereto.

In the present invention, the antibody or single-chain variable fragment thereof may be selected from the group consisting of SEQ ID NOs: 1 to 3, but is not limited thereto.

In one embodiment, the single-chain variable fragment may be a KRAS mutant scFv represented by SEQ ID NO: 1 below.

SEQ ID NO: 1

MAWVWTLLFLMAAAQSIQAEWIKQRPGQGLEWIGVIHPGNGGTNYNENFKG

KATLTADKSSSTAYMQLSSLTSDDSAVYFCASGNDGSYWGQGTTVTVSSGGGGSGKGGS

GGGGSGGGGSDIELTQSPSSLTVTAGEKVTMSCKSSQSLLNSGDQKIYLTWYQQKPGQP

PKLLIYWASTRESGVPDRFTGSVSGTDFTLTISSVQAEDLAVYYCQNDYNYPYTFGGGT

KLEIKRGSENLYFQGGSGKPIPNPLLGLDSTGGSGGSHHHHHH

Here, MAWVWTLLFLMAAAQSIQA is a signal peptide, the amino acid sequence following the signal peptide is a variable heavy chain (V_{H}) region, GGGGSGKGGSGGGGSGGGGS is a linker, and the amino acid sequence following the linker is a variable light chain (V_{L}) region. The signal peptide may also be replaced with a known sequence such as MTRLTVLALLAGLLASSRA or MKWVTFISLLFLFSSAYS.

In another embodiment, the single-chain variable fragment may be a KRAS mutant scFv represented by SEQ ID NO: 2 below.

SEQ ID NO: 2

MAQVKLQESGPELVRPGTSVKVSCKASGYAFTNYLIEWIKQRPGQGLEWIG

VIHPGNGGTNYNENFKGKATLTADKSSSTAYMQLSSLTSDDSAVYFCASGNDGSYWGQG

TTVTVSSGGGGSGGGGSGGGGSDIELTQSPSSLTVTAGEKVTMSCKSSQSLLNSGDQKI

YLTWYQQKPGQPPKLLIYWASTRESGVPDRFTGSVSGTDFTLTISSVQAEDLAVYYCQN

DYNYPYTFGGGTKLEIKRLEHHHHHH

MAQVKLQESGPELVRPGTSVKVSCKASGYAFTNYLI is a signal peptide, the amino acid sequence following the signal peptide is a variable heavy chain (V_{H}) region, GGGGSGGGGSGGGGS is a linker, and the amino acid sequence following the liker is a variable light chain (V_{L}) region.

In another embodiment, the single-chain variable fragment may be a KRAS mutant scFv represented by SEQ ID NO: 3 below.

SEQ ID NO: 3

MAWVWTLLFLMAAAQSIQAEWIKQRPGQGLEWIGVIHPGNGGTNYNENFKG

KATLTADKSSSTAYMQLSSLTSDDSAVYFCASGNDGSYWGQGTTVTVSSGGGGSGKGGS

GGGGSGGGGSDIELTQSPSSLTVTAGEKVTMSCKSSQSLLNSGDQKIYLTWYQQKPGQP

PKLLIYWASTRESGVPDRFTGSVSGTDFTLTISSVQAEDLAVYYCQNDYNYPYTFGGGT

KCLEIKRGSENLYFQGGSGKPIPNPLLGLDSTGGSGGSHHHHHH

SEQ ID NO: 3 is the same as SEQ ID NO: 1, except that cysteine is introduced after V_{L}.

In the present invention, the cancer cell-penetrating peptide may be selected from the group consisting of CCPP1 (H4K), CCPP2 (H4P), CCPP3 (LMWP) and CCPP4 (hBD3-3), but is not limited thereto. The cancer cell-penetrating peptide may be selected from the group consisting of SEQ ID NO: 86 to SEQ ID NO: 89, but is not limited thereto.

In the present invention, the cancer cell-penetrating may be linked to the N-terminus or C-terminus of the antibody or single-chain variable fragment thereof via a linker.

In the present invention, the linker serves to provide a space so that the cancer cell-penetrating peptide and the antibody or single-chain variable fragment thereof may individually form a functional structure when linked together. The linker is preferably a peptide linker comprising an appropriate combination of amino acids G and S. Here, more preferably, the number of amino acid residues G is from 3 to 20, and the number of amino acid residues S is from 1 to 5, but the present invention is not limited thereto. Most preferably, the linker is GGGGS or GGGGSGGGGSGGGGS.

In the present invention, when the linker and the cancer cell-penetrating peptide are linked together, they may be represented by the amino acid sequence of any one of SEQ ID NO: 4 to SEQ ID NO: 11, without being limited thereto. In this case, the fusion peptide represented by any one of the amino acid sequences of SEQ ID NOs: 4 to 7 is preferably linked to the C terminus of the single-chain variable fragment, and the fusion peptide represented by the amino acid sequence of any one of SEQ ID NOs: 8 to 11 is preferably linked to the N-terminus of the single-chain variable fragment. In another embodiment, the fusion peptide represented by the amino acid sequence of any one of SEQ ID NOs: 4 to 11 may be further bound to a linker connecting the heavy chain to the light chain of the single-chain fragment of the antibody. Meanwhile, when the fusion peptide represented by any one of the amino acid sequences of SEQ ID NOs: 4 to 11 is linked to the antibody, it is preferably linked to a sugar structure present in the Fc region.

In the present invention, the antibody or single-chain variable fragment thereof and the cancer cell-penetrating peptide may be linked together by a chemical conjugation method. In this case, the fusion protein may be represented by the amino acid sequence of any one of SEQ ID NOs: 68 to 83, but is not limited thereto.

In the present invention, the fusion protein may be one wherein the cancer cell-penetrating peptides of any one selected from the group consisting of SEQ ID NOs: 86 to 89 is linked via a linker to the lysine or cysteine residue of the single-chain variable fragment of any one selected from the group consisting of SEQ ID NOs: 1 to 3.

In the present invention, the linker may be CGGGGG or CGGGGGSSGGGGG.

In the present invention, the antibody or single-chain variable fragment thereof and the cancer cell-penetrating peptide may be linked together and expressed by a gene expression method. In this case, the fusion protein may be expressed in *Escherichia coli* and may be represented by the amino acid sequence of any one of SEQ ID NOs: 12 to 27, without being limited thereto. In addition, the fusion protein may be expressed in mammalian cells, and may be represented by the amino acid sequence of any one of SEQ ID NO: 28 to SEQ ID NO: 59, and SEQ ID NO: 90 to SEQ ID NO: 93, without being limited thereto.

For example, the cancer cell-penetrating peptide and the linker may be linked to the N-terminus of the single-chain variable fragment (any one of SEQ ID NO: 1 to SEQ ID NO: 3) which targets the KRAS mutant protein, or the cancer cell-penetrating peptide and the linker may be linked to the N-terminus of the single-chain variable fragment. Various embodiments related thereto are specifically described in Tables 2 to 5 in Examples below.

In the present invention, the fusion protein may be represented by any one of the amino acid sequences of SEQ ID NO: 12 to SEQ ID NO: 59, SEQ ID NO: 68 to SEQ ID NO: 83, and SEQ ID NO: 90 to SEQ ID NO: 93.

In the present invention, the fusion protein may be produced by a gene expression method or a chemical conjugation method, but the production method is not limited thereto.

When the fusion protein is produced by the gene expression method, it is possible to use a vector that expresses both (i) the antibody or single-chain variable fragment thereof that targets the intracellular oncogenic protein or oncogenic mutant protein, and (ii) the cancer cell-penetrating peptide. In this case, the vector for expression may further contain a linker sequence between the antibody or single-chain variable fragment thereof and the cancer cell-penetrating peptide. A pET vector, pcDNA 3.4, pcDNA3.1, pcDNA3.1-TOPO, pcDNA3.4-TOPO, pSecTag vector, etc. may be used, but the vector is not limited thereto. In the present invention, a pET vector was used for expression in *E. coli,* and a pcDNA3.1-TOPO or pcDNA3.4-TOPO vector was used for expression in mammalian cells.

Therefore, in another aspect, the present invention is directed to a nucleic acid encoding the fusion protein.

The present invention is also directed to a recombinant vector into which the nucleic acid has been introduced.

The present invention is also directed to a recombinant cell into which the recombinant vector has been introduced.

In the present invention, the recombinant cell may be an *E. coli* or mammalian cell, but is not limited thereto.

Examples of preferred mammalian cell lines that may be used in the present invention include, but are not limited to, Chinese hamster ovarian (CHO) cells, human embryonic kidney cells (HEK293), and the like.

The present invention is also directed to a method for producing the fusion protein comprising steps of: (a) expressing the fusion protein by culturing the recombinant cell; and (b) recovering the expressed fusion protein.

Meanwhile, in the present invention, the antibody or single-chain variable fragment thereof that targets the intracellular oncogenic protein or oncogenic mutant protein may be expressed and purified, and then the cancer cell-penetrating peptide may be introduced thereto by a chemical conjugation method.

The cancer cell-penetrating peptide may be linked to the antibody or single-chain variable fragment thereof via a linker and a crosslinker. As the linker, any linker may be used as long as it may provide a space capable of forming a functional structure. For example, the linker may be a peptidic linker of natural and/or synthetic origin. The peptidic linker of natural and/or synthetic origin may consist of an amino acid chain consisting of 1 to 50 amino acids, and may comprise repetitive amino acid sequences or sequences of naturally occurring polypeptides, such as polypeptides with a hinge-function. In another embodiment, the peptidic linker amino acid sequence may be a "synthetic linker amino acid sequence" that is designated to be rich in glycine, glutamine, and/or serine residues. These residues may be arranged in small repetitive units of up to five amino acids, and the small repetitive unit may be repeated to form a multimeric unit. At the amino- and/or carboxy-terminal ends of the multimeric unit, up to six additional arbitrary, naturally occurring amino acids may be added. Other synthetic peptidic linkers may be composed of a single amino acid, which is repeated between 10 to 20 times, and may comprise, at the amino- and/or carboxy-terminal end, up to six additional arbitrary, naturally occurring amino acids. Meanwhile, the linker may be in a form in which amino acids are chemically modified. For example, the linker may be in the form of Fmoc-6-aminohexanoic acid to which Fmoc-(9-fluorenylmethoxycarbonyl) as a blocking group is bonded, but the linker is not limited thereto.

In some embodiments of the present invention, the fusion peptide in which the cancer cell-penetrating peptide and the linker are linked together may be represented by the amino acid sequence of any one of SEQ ID NOs: 60 to 67. In this case, the sulfhydryl group of cysteine located at the N-terminus of the cancer cell-penetrating peptide may be linked to the amine group of lysine in the linker moiety of SEQ ID NO: 1 or the amine group of lysine in the exposed portion in the three-dimensional structure.

In this case, examples of the crosslinker that may be used include, but are not limited to, 1,4-bis-maleimidobutane (BMB), 1,11-bis-maleimidotetraethyleneglycol (BM[PEO]4), 1-ethyl-3-[3-dimethyl aminopropyl]carbodiimide hydrochloride (EDC), succinimidyl-4-[N-maleimidomethylcyclohexane-1-carboxy-[6-amidocaproate] (SMCC) and its sulfonate (sulfo-SMCC), succinimidyl 6-[3-(2-pyridyldithio)-propionamide]hexanoate (SPDP) and its sulfonate (sulfo-SPDP), m-maleimidobenzoyl-N-hydroxysuccinimide ester (MBS) and its sulfonate (sulfo-MBS), succinimidyl [4-(p-maleimidophenyl)butyrate] (SMPB) and its sulfonate (sulfo-SMPB), etc.

Also, the crosslinker, which may be linked with the sulfhydryl group of cysteine located at the N-terminus of the fusion peptide represented by any one amino acid sequence represented by SEQ ID NOs: 60 to 67 after reducing the sulfhydryl group of cysteine at the C-terminus of SEQ ID NO: 3, may be tris(2-carboxyethyl)phosphine (TCEP), 5,50-dithiobis-(2-nitrobenzoic acid (DTNB), etc., but is not limited thereto.

In the present invention, the method of producing the fusion protein using the chemical conjugation method comprises steps of:
(a) activating the amine group of lysine, which is present in the linker moiety or exposed portion of the antibody or single-chain variable fragment thereof which targets the intracellular oncogenic protein or oncogenic mutant protein, with a crosslinker;
(b) linking the cancer cell-penetrating peptide to the antibody or single-chain variable fragment thereof activated with the crosslinker; and
(c) purifying the fusion protein wherein the cancer cell-penetrating peptide is linked to the antibody or single-chain variable fragment thereof.

Alternatively, step (a) of the above method may be a step of reducing the sulfhydryl group of the cysteine residue of the antibody or single-chain variable fragment thereof which targets the intracellular oncogenic protein or oncogenic mutant protein.

The fusion protein produced by the chemical conjugation method may be represented by the amino acid sequence of any one of SEQ ID NOs: 68 to 83, but is not limited thereto.

In an embodiment of the present invention, the fusion protein may be one wherein the cancer cell-penetrating peptide is linked via a crosslinker to the lysine or cysteine residue of the antibody or single-chain variable fragment thereof which targets the intracellular oncogenic protein or oncogenic mutant protein.

Preferably, the fusion protein may be one wherein the fusion peptide selected from the group consisting of SEQ ID NOs: 60 to 67 is chemically bound to the lysine or cysteine residue of any one single-chain variable fragment selected from the group consisting of SEQ ID NOs: 1 to 3 by a crosslinker.

In this case, wherein the residue is lysine, the crosslinker may be selected from the group consisting of 1,4-bis-maleimidobutane (BMB), 1,11-bis-maleimidotetraethyleneglycol (BM[PEO]4), 1-ethyl-3-[3-dimethyl aminopropyl]carbodiimide hydrochloride (EDC), succinimidyl-4-[N-maleimidomethylcyclohexane-1-carboxy-[6-amidocaproate]] (SMCC) and its sulfonate (sulfo-SMCC), succinimidyl 6-[3-(2-pyridyldithio)-propionamide]hexanoate (SPDP) and its sulfonate (sulfo-SPDP), m-maleimidobenzoyl-N-hydroxysuccinimide ester (MBS) and its sulfonate (sulfo-MBS), and succinimidyl [4-(p-maleimidophenyl)butyrate] (SMPB) and its sulfonate (sulfo-SMPB).

Meanwhile, when the residue is cysteine, the crosslinker may be tris(2-carboxyethyl)phosphine (TCEP) or 5,50-dithiobis-(2-nitrobenzoic acid (DTNB).

The KRAS mutant protein according to the present invention is known as an important factor in tumorigenesis that accelerates the growth of cancer cells, but exists inside the cell membrane, and thus the antibody or single-chain variable fragment thereof that targets the KRAS mutant protein has no significant inhibitory effect on the KRAS mutant protein. However, as a result of evaluating the tumor growth inhibitory effect of the KRAS mutant-targeting antibody or single-chain variable fragment thereof after introducing the cancer cell-penetrating peptide thereto, it was confirmed from the results of an *in vitro* experiment and an animal experiment that the KRAS mutant-targeting antibody or single-chain variable fragment having the cancer cell-penetrating ability had an effect of significantly inhibiting the growth of tumor cells.

Therefore, in another aspect, the present invention is directed to a pharmaceutical composition for treating a tumor comprising the fusion protein as an active ingredient.

In the present invention, the tumor may be at least one selected from the group consisting of non-small cell lung cancer, colorectal cancer, pancreatic cancer, bladder cancer, kidney cancer, thyroid cancer, breast cancer, colon cancer, liver cancer, brain tumor, skin cancer, melanoma, colorectal cancer, prostate cancer, and blood cancer, but is not limited thereto.

In the present invention, the pharmaceutical composition may be formulated in any one form selected from the group consisting of liquid formulations (for example, for injection) such as injections, formulations for oral administration, aqueous solutions, suspensions and emulsions, capsules, granules, tablets, and formulations for mucosal administration, but is not limited thereto. These formulations may be prepared by conventional methods used for formulation in the art or by methods disclosed in Remington's Pharmaceutical Science (latest edition), Mack Publishing Company, Easton PA, and may vary depending on each disease or components.

Meanwhile, the pharmaceutical composition of the present invention may further comprise at least one pharmaceutically acceptable carrier, in addition to the antibody or single-chain variable fragment thereof that has the ability to penetrate cancer cells and targets the KRAS mutant protein. The pharmaceutically acceptable carrier may be at least one selected from among saline, sterile water, Ringer's solution, buffered saline, dextrose solution, maltodextrin solution, glycerol, ethanol, and combinations thereof.

The pharmaceutical composition of the present invention may further comprise a pharmaceutically acceptable adjuvant, if necessary. The adjuvant may be one or more selected from the group consisting of excipients, diluents, dispersants, buffers, antimicrobial preservatives, bacteriostatic agents, surfactants, binders, lubricants, antioxidants, thickeners, and viscosity modifiers, but is not limited thereto.

The pharmaceutical composition according to the present invention may be administered orally or parenterally (e.g., intravenously, subcutaneously, intraperitoneally or topically) according to a desired method, and the dosage thereof may vary depending on the patient's weight, age, sex, health status and diet, the time of administration, the mode of administration, excretion rate, and the severity of disease and may be determined by an expert.

In one embodiment of the present invention, the single dose of the peptide may be 1 µg/kg to 100 mg/kg, preferably 5 µg/kg to 50 mg/kg, and may be administered once a day or 1 to 3 times a week, but the dose and administration frequency are not limited thereto

In another aspect, the present invention is directed to a method for preventing or treating a tumor comprising a step of administering the fusion protein.

In another aspect, the present invention is directed to the use of the fusion protein for preventing or treating a tumor.

In another aspect, the present invention is directed to the use of the fusion protein in the manufacture of a medicament for treating a tumor.

### Examples

Hereinafter, the present invention will be described in more detail with reference to examples. These examples serve merely to illustrate the present invention, and it will be obvious to those of ordinary skill in the art that the scope of the present invention is not to be construed as being limited by these examples.

### Example 1: Preparation of Cancer Cell-Penetrating and KRAS Mutant-Targeting scFv by Protein Expression Method

### Example 1-1: Expression of Cancer Cell-Penetrating and KRAS Mutant-Targeting scFv in E. coli

KRAS mutant scFv expression vectors, each comprising a KRAS mutant scFv and a cancer cell-penetrating peptide, were prepared by a PCR method using specific primers. The KRAS mutant scFv used in the present invention is any one of SEQ ID NOs: 1 to 3.
KRAS mutant scFv (SEQ ID NO: 1): 
MAWVWTLLFLMAAAQSIQAEWIKQRPGQGLEWIGVIHPGNGGTNYNENFKG
KATLTADKSSSTAYMQLSSLTSDDSAVYFCASGNDGSYWGQGTTVTVSSGGGGSGKGGS
GGGGSGGGGSDIELTQSPSSLTVTAGEKVTMSCKSSQSLLNSGDQKIYLTWYQQKPGQP
PKLLIYWASTRESGVPDRFTGSVSGTDFTLTISSVQAEDLAVYYCQNDYNYPYTFGGGT
KLEIKRGSENLYFQGGSGKPIPNPLLGLDSTGGSGGSHHHHHH
KRAS mutant scFv (SEQ ID NO: 2): 
MAQVKLQESGPELVRPGTSVKVSCKASGYAFTNYLIEWIKQRPGQGLEWIG
VIHPGNGGTNYNENFKGKATLTADKSSSTAYMQLSSLTSDDSAVYFCASGNDGSYWGQG
TTVTVSSGGGGSGGGGSGGGGSDIELTQSPSSLTVTAGEKVTMSCKSSQSLLNSGDQKI
YLTWYQQKPGQPPKLLIYWASTRESGVPDRFTGSVSGTDFTLTISSVQAEDLAVYYCQN
DYNYPYTFGGGTKLEIKRLEHHHHHH
KRAS mutant scFv (SEQ ID NO: 3):

[157]    MAWVWTLLFLMAAAQSIQAEWIKQRPGQGLEWIGVIHPGNGGTNYNENFKG
KATLTADKSSSTAYMQLSSLTSDDSAVYFCASGNDGSYWGQGTTVTVSSGGGGSGKGGS
GGGGSGGGGSDIELTQSPSSLTVTAGEKVTMSCKSSQSLLNSGDQKIYLTWYQQKPGQP
PKLLIYWASTRESGVPDRFTGSVSGTDFTLTISSVQAEDLAVYYCQNDYNYPYTFGGGT
KCLEIKRGSENLYFQGGSGKPIPNPLLGLDSTGGSGGSHHHHHH

pET 21a(+) DNA was digested with Nde I and Xho I, and pMX DNA was also digested with Nde I and Xho I. The 767-bp Kras scFv gene fragment was recovered by an agarose electroelution method. To insert the gene fragment into a pET 21a(+) vector, the gene was amplified by PCR. The PCR was performed using the forward primer (5'-AAGGAGATATACATATGATGGCATGGGTTTGGAC-3') and the reverse primer (5'-AGCCCGAAGGGAATTCATTTGCAGATACAAAGTGTTTTTAGAGTTG-3'). Each digested vector and insert were mixed together at a ratio of 0.5 µg: 1.0 µg, and ligated together using T4 DNA ligase in a ligation buffer comprising 500 mM Tris-HCl, 100 mM MgCl₂, 200 mM DTT and 10 mM ATP at 4°C for 16 to 18 hours.

The recombinant DNA solution was transformed into E. *coli* DH5a and plated on LB+Amp solid medium, and transformants were selected.

A single colony of an *E*. *coli* Rosetta^{™} 2(DE3) Singles^{™} (Novagen) strain transformed with each plasmid was inoculated into 20 ml of LB comprising 10 µg/ml of ampicillin antibiotic, and cultured at 37°C overnight. 1 ml of the culture was diluted in 200 ml of fresh LB comprising the same concentration of the antibiotic. The expression of KRAS scFv was induced by adding IPTG to a final concentration of 1 mM to the cells at an OD600 value of 0.8, followed by culturing overnight. The cells were harvested through centrifugation and lysed using 10 ml lysis buffer (50 mM NaH₂PO₄, 300 mM NaCl, pH 8.0). After sonication of the cells, the protein was separated into total, soluble and insoluble fractions by centrifugation at 12,000g and 4°C for 10 minutes, and analyzed by SDS-PAGE (S.I. Choi et al., Protein solubility and folding enhancement by interaction with RNA, PLoS ONE 3 (2008) e2677).

The isolated KRAS scFv was separated and purified by nickel chromatography (HisTrap^{™} excel Kit, GE Healthcare). Next, the nickel column was equilibrated with a buffer [50 mM NaH₂PO₄ (pH 8.0), 300 mM NaCl], and then an aqueous solution of the protein in the same buffer was applied to the column. After washing with the above containing 20 mM imidazole, the protein was separated using a buffer containing imidazole that was gradually increased from 50 mM to 500 mM. The separated proteins were collected and dialyzed using a desalting column (PD-10 Columns, GE Healthcare).

The linker and cancer cell-penetrating peptide sequences used in the present invention are shown in Table 1 below.

**[Table 1]**

| Type and position of linker | Cancer cell-penetrating peptide (CCPP) | Amino acid sequences of linker and cancer cell-penetrating peptide | SEQ ID NO | Linking portion of scFv |
|---|---|---|---|---|
| N terminus of CCPP-GGGGS | CCPP-1 (H4K) | GGGGS-HRRCNKNNKKR | 4 | C terminus |
| | CCPP-2 (H4P) | GGGGS-HRRCNPNNKKR | 5 | C terminus |
| | CCPP-3 (LMWP) | GGGGS-VSRRRRRRGGRRRR | 6 | C terminus |
| | CCPP-4 (hBD3-3) | GGGGS-GKCSTRGRKCCRRKK | 7 | C terminus |
| C terminus of CCPP-GGGGS | CCPP-1 (H4K) | HRRCNKNNKKR-GGGGS | 8 | N terminus |
| | CCPP-2 (H4P) | HRRCNPNNKKR-GGGGS | 9 | N terminus |
| | CCPP-3 (LMWP) | VSRRRRRRGGRRRR-GGGGS | 10 | N terminus |
| | CCPP-4 (hBD3-3) | GKCSTRGRKCCRRKK-GGGGS | 11 | N terminus |

Table 2 below shows the amino acid sequences of the fusion proteins for *E. coli* expression wherein the cancer cell-penetrating peptide and the KRAS mutant scFv are linked together.

**[Table 2]**

| Fusion protein | Amino acid sequence | SEQ ID NO |
|---|---|---|
| SEQ ID NO 1-GGGGS-H4K | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT | 12 |
| | TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - HRRCNKNNKKR - LEIKRGSENLYFQGGSGKPIPNPLLGLDSTGGSGGSHHHHHH | |
| SEQ ID NO 1-GGGGS-H4P | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - HRRCNPNNKKR - LEIKRGSENLYFQGGSGKPIPNPLLGLDSTGGSGGSHHHHHH | 13 |
| SEQ ID NO 1-GGGGS-LMWP | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - VSRRRRRRGGRRRR- LEIKRGSENLYFQGGSGKPIPNPLLGLDSTGGSGGSHHHHHH | 14 |
| SEQ ID NO 1-GGGGS-hBD3-3 | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - | 15 |
| | GGGGS          -          GKCSTRGRKCCRRKK LEIKRGSENLYFQGGSGKPIPNPLLGLDSTGGSGGSHHHHHH | |
| H4K-GGGGS-SEQ ID NO 1 | MAWVWTLLFLMAAAQSIQA - HRRCNKNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | 16 |
| H4P-GGGGS-SEQ ID NO 1 | MAWVWTLLFLMAAAQSIQA - HRRCNPNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | 17 |
| LMWP-GGGGS-SEQ ID NO 1 | MAWVWTLLFLMAAAQSIQA- VSRRRRRRGGRRRR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | 18 |
| hBD3-3-GGGGS-SEQ ID NO 1 | MAWVWTLLFLMAAAQSIQA - GKCSTRGRKCCRRKK - GGGGS - EWIKQRPGQGLEWIGVIHPGNGGTNYNENFKGKATLTADKSSSTAYM | 19 |
| | QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | |
| SEQ ID NO 2-GGGGS-H4K | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - HRRCNKNNKKR - LEIKRLEHHHHHH | 20 |
| SEQ ID NO 2-GGGGS-H4P | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - HRRCNPNNKKR - LEIKRLEHHHHHH | 21 |
| SEQ ID NO 2-GGGGS-LMWP | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - VSRRRRRRGGRRRR -LEIKRLEHHHHHH | 22 |
| SEQ ID NO 2-GGGGS-hBD3-3 | MAQVKLQESGPELVRPGTSVKVSCKASGYAFTNYLIEWIKQRPGQGL EWIGVIHPGNGGTNYNENFKGKATLTADKSSSTAYMQLSSLTSDDSA | 23 |
| | VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - GKCSTRGRKCCRRKK - LEIKRLEHHHHHH | |
| H4K-GGGGS-SEQ ID NO 2 | MAQVKLQESGPELVRPGTSVKVSCKASGYAFTNYLI - HRRCNKNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 24 |
| H4P-GGGGS-SEQ ID NO 2 | MAQVKLQESGPELVRPGTSVKVSCKASGYAFTNYLI - HRRCNPNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 25 |
| LMWP-GGGGS-SEQ ID NO 2 | MAQVKLQESGPELVRPGTSVKVSCKASGYAFTNYLI- VSRRRRRRGGRRRR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 26 |
| hBD3-3-GGGGS-SEQ ID NO 2 | MAQVKLQESGPELVRPGTSVKVSCKASGYAFTNYLI - GKCSTRGRKCCRRKK - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 27 |

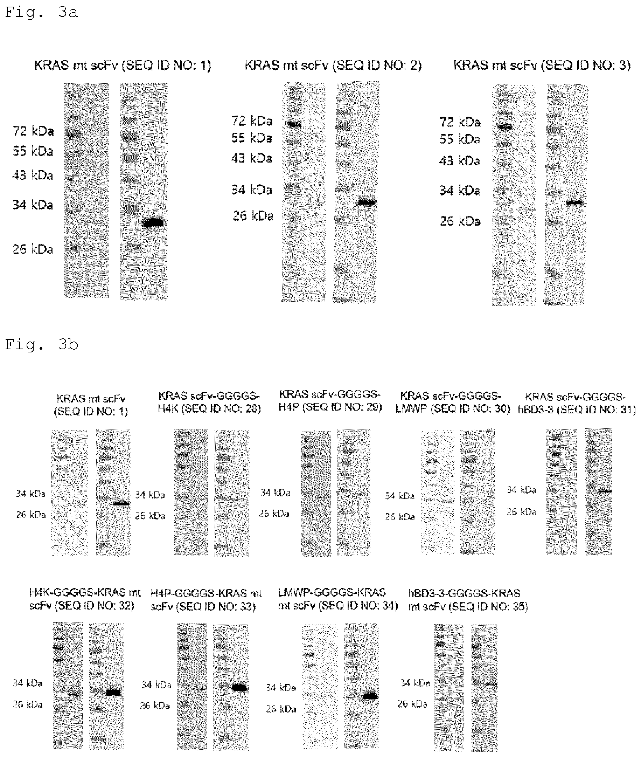

FIG. 3(A) shows the results of SDS-PAGE and Western blot analysis of the KRAS mutant-targeting purified antibodies or single-chain variable fragments of SEQ ID NOs: 1, 2 and 3.

### Example 1-2: Expression of Cancer Cell-Penetrating and KRAS Mutant-Targeting scFv in Mammalian Cells

KRAS mutant scFv expression vectors (each containing a KRAS mutant scFv and a cancer cell-penetrating peptide) for use in mammalian cells were constructed by a PCR method using specific primers.

Table 3 below shows the amino acid sequences of the fusion proteins wherein the cancer cell-penetrating peptide is linked to the KRAS mutant scFv and which are each cloned into the pcDNA3.4-TOPO vector for mammalian cell expression.

**[Table 3]**

| Fusion protein | Amino acid sequence | SEQ ID NO |
|---|---|---|
| SEQ ID NO 1-GGGGS-H4K | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - HRRCNKNNKKR - LEIKRGSENLYFQGGSGKPIPNPLLGLDSTGGSGGSHHHHHH | 28 |
| SEQ ID NO 1-GGGGS-H4P | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - HRRCNPNNKKR - LEIKRGSENLYFQGGSGKPIPNPLLGLDSTGGSGGSHHHHHH | 29 |
| SEQ ID NO 1-GGGGS-LMWP | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - VSRRRRRRGGRRRR- LEIKRGSENLYFQGGSGKPIPNPLLGLDSTGGSGGSHHHHHH | 30 |
| SEQ ID NO 1-GGGGS-hBD3-3 | MAWVWTLLFLMAAAQSIQAEWIKQRPGQGLEWIGVIHPGNGGTNYNE NFKGKATLTADKSSSTAYMQLSSLTSDDSAVYFCASGNDGSYWGQGT TVTVSSGGGGSGKGGSGGGGSGGGGSDIELTQSPSSLTVTAGEKVTM SCKSSQSLLNSGDQKIYLTWYQQKPGQPPKLLIYWASTRESGVPDRF TGSVSGTDFTLTISSVQAEDLAVYYCQNDYNYPYTFGGGTK - GGGGS - GKCSTRGRKCCRRKK - LEIKRGSENLYFQGGSGKPIPNPLLGLDSTGGSGGSHHHHHH | 31 |
| H4K-GGGGS-SEQ ID NO 1 | MAWVWTLLFLMAAAQSIQA - HRRCNKNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | 32 |
| H4P-GGGGS-SEQ ID NO 1 | MAAWVWTLLFLMAAAQSIQA - HRRCNPNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | 33 |
| LMWP-GGGGS-SEQ ID NO 1 | MAWVWTLLFLMAAAQSIQA- VSRRRRRRGGRRRR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT | 34 |
| | WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | |
| hBD3-3-GGGGS-SEQ ID NO 1 | MAWVWTLLFLMAAAQSIQA - GKCSTRGRKCCRRKK - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRGSENLYFQGGSGKPIPNPLL GLDSTGGSGGSHHHHHH | 35 |
| SEQ ID NO 2-GGGGS-H4K | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - HRRCNKNNKKR - LEIKRLEHHHHHH | 36 |
| SEQ ID NO 2-GGGGS-H4P | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - HRRCNPNNKKR - LEIKRLEHHHHHH | 37 |
| SEQ ID NO 2-GGGGS-LMWP | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS | 38 |
| | SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - VSRRRRRRGGRRRR -LEIKRLEHHHHHH | |
| SEQ ID NO 2-GGGGS-hBD3-3 | MAQVKLQESGPELVRPGTSVKVSCKASGYAFTNYLIEWIKQRPGQGL EWIGVIHPGNGGTNYNENFKGKATLTADKSSSTAYMQLSSLTSDDSA VYFCASGNDGSYWGQGTTVTVSSGGGGSGGGGSGGGGSDIELTQSPS SLTVTAGEKVTMSCKSSQSLLNSGDQKIYLTWYQQKPGQPPKLLIYW ASTRESGVPDRFTGSVSGTDFTLTISSVQAEDLAVYYCQNDYNYPYT FGGGTK - GGGGS - GKCSTRGRKCCRRKK - LEIKRLEHHHHHH | 39 |
| H4K-GGGGS-SEQ ID NO 2 | MAQVKLQESGPELVRPGTSVKVSCKASGYAFTNYLI - HRRCNKNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 40 |
| H4P-GGGGS-SEQ ID NO 2 | MAQVKLQESGPELVRPGTSVKVSCKASGYAFTNYLI - [?] HRRCNPNNKKR - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 41 |
| LMWP-GGGGS-SEQ ID NO 2 | MAQVKLQESGPELVRPGTSVKVSCKASGYAFTNYLI- VSRRRRRRGGRRRR - GGGGS - | 42 |
| | EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | |
| hBD3-3-GGGGS-SEQ ID NO 2 | MAQVKLQESGPELVRPGTSVKVSCKASGYAFTNYLI - GKCSTRGRKCCRRKK - GGGGS - EWIKQRPGQGLEWIGVIHPGNGGTNYNENFKGKATLTADKSSSTAYM QLSSLTSDDSAVYFCASGNDGSYWGQGTTVTVSSGGGGSGKGGSGGG GSGGGGSDIELTQSPSSLTVTAGEKVTMSCKSSQSLLNSGDQKIYLT WYQQKPGQPPKLLIYWASTRESGVPDRFTGSVSGTDFTLTISSVQAE DLAVYYCQNDYNYPYTFGGGTKLEIKRLEHHHHHH | 43 |

Table 4 below shows the amino acid sequences of the fusion proteins wherein the cancer cell-penetrating peptide is linked to the KRAS mutant scFv and which are each cloned into the pcDNA3.1-TOPO vector for mammalian cell expression.

**[Table 4]**

| Fusion protein | Amino acid sequence | SEQ ID NO |
|---|---|---|
| SEQ ID NO 1-GGGGS-H4K | MAWVWTLLFLMAAAQSIQAEWIKQRPGQGLEWIGVIHPGNGGTNYNENFKGK ATLTADKSSSTAYMQLSSLTSDDSAVYFCASGNDGSYWGQGTTVTVSSGGGG SGKGGSGGGGSGGGGSDIELTQSPSSLTVTAGEKVTMSCKSSQSLLNSGDQK IYLTWYQQKPGQPPKLLIYWASTRESGVPDRFTGSVSGTDFTLTISSVQAED | 44 |
| | LAVYYCQNDYNYPYTFGGGTK - GGGGS - HRRCNKNNKKR - LEIKRGSENLYFQGGSGKPIPNPLLGLDSTGGSGGSHHHHHH | |
| SEQ ID NO 1-GGGGS-H4P | MAWVWTLLFLMAAAQSIQAEWIKQRPGQGLEWIGVIHPGNGGTNYNENFKGK ATLTADKSSSTAYMQLSSLTSDDSAVYFCASGNDGSYWGQGTTVTVSSGGGG SGKGGSGGGGSGGGGSDIELTQSPSSLTVTAGEKVTMSCKSSQSLLNSGDQK IYLTWYQQKPGQPPKLLIYWASTRESGVPDRFTGSVSGTDFTLTISSVQAED LAVYYCQNDYNYPYTFGGGTK - GGGGS - HRRCNPNNKKR - LEIKRGSENLYFQGGSGKPIPNPLLGLDSTGGSGGSHHHHHH | 45 |
| SEQ ID NO 1-GGGGS-LMWP | MAWVWTLLFLMAAAQSIQAEWIKQRPGQGLEWIGVIHPGNGGTNYNENFKGK ATLTADKSSSTAYMQLSSLTSDDSAVYFCASGNDGSYWGQGTTVTVSSGGGG SGKGGSGGGGSGGGGSDIELTQSPSSLTVTAGEKVTMSCKSSQSLLNSGDQK IYLTWYQQKPGQPPKLLIYWASTRESGVPDRFTGSVSGTDFTLTISSVQAED LAVYYCQNDYNYPYTFGGGTK - GGGGS - VSRRRRRRGGRRRR- LEIKRGSENLYFQGGSGKPIPNPLLGLDSTGGSGGSHHHHHH | 46 |
| SEQ ID NO 1-GGGGS-hBD3-3 | MAWVWTLLFLMAAAQSIQAEWIKQRPGQGLEWIGVIHPGNGGTNYNENFKGK ATLTADKSSSTAYMQLSSLTSDDSAVYFCASGNDGSYWGQGTTVTVSSGGGG SGKGGSGGGGSGGGGSDIELTQSPSSLTVTAGEKVTMSCKSSQSLLNSGDQK IYLTWYQQKPGQPPKLLIYWASTRESGVPDRFTGSVSGTDFTLTISSVQAED LAVYYCQNDYNYPYTFGGGTK - GGGGS - GKCSTRGRKCCRRKK - LEIKRGSENLYFQGGSGKPIPNPLLGLDSTGGSGGSHHHHHH | 47 |
| H4K-GGGGS-SEQ ID NO 1 | MAWVWTLLFLMAAAQSIQA - HRRCNKNNKKR - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY | 48 |
| | WASTRESGVPDRFTGSVSGTDFTLTISSVQAEDLAVYYCQNDYNYPYTFGGG TKLEIKRGSENLYFQGGSGKPIPNPLLGLDSTGGSGGSHHHHHH | |
| H4P-GGGGS-SEQ ID NO 1 | MAWVWTLLFLMAAAQSIQA - HRRCNPNNKKR - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY WASTRESGVPDRFTGSVSGTDFTLTISSVQAEDLAVYYCQNDYNYPYTFGGG TKLEIKRGSENLYFQGGSGKPIPNPLLGLDSTGGSGGSHHHHHH | 49 |
| LMWP-GGGGS-SEQ ID NO 1 | MAWVWTLLFLMAAAQSIQA-   VSRRRRRRGGRRRR   -   GGGGS   - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY WASTRESGVPDRFTGSVSGTDFTLTISSVQAEDLAVYYCQNDYNYPYTFGGG TKLEIKRGSENLYFQGGSGKPIPNPLLGLDSTGGSGGSHHHHHH | 50 |
| hBD3-3-GGGGS-SEQ ID NO 1 | MAWVWTLLFLMAAAQSIQA - GKCSTRGRKCCRRKK - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY WASTRESGVPDRFTGSVSGTDFTLTISSVQAEDLAVYYCQNDYNYPYTFGGG TKLEIKRGSENLYFQGGSGKPIPNPLLGLDSTGGSGGSHHHHHH | 51 |
| SEQ ID NO 2-GGGGS-H4K | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD | 52 |
| | FTLTISSVQAEDLAVYYCQNDYNYPYTFGGGTK - GGGGS HRRCNKNNKKR - LEIKRLEHHHHHH | |
| SEQ ID NO 2-GGGGS-H4P | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD FTLTISSVQAEDLAVYYCQNDYNYPYTFGGGTK - GGGGS - HRRCNPNNKKR - LEIKRLEHHHHHH | 53 |
| SEQ ID NO 2-GGGGS-LMWP | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD FTLTISSVQAEDLAVYYCQNDYNYPYTFGGGTK - GGGGS - VSRRRRRRGGRRRR -LEIKRLEHHHHHH | 54 |
| SEQ ID NO 2-GGGGS-hBD3-3 | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD FTLTISSVQAEDLAVYYCQNDYNYPYTFGGGTK - GGGGS - GKCSTRGRKCCRRKK - LEIKRLEHHHHHH | 55 |
| H4K-GGGGS-SEQ ID NO 2 | MAQVKLQESGPELVRPGTSVKVSCKASGYAFTNYLI - HRRCNKNNKKR - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY | 56 |
| | WASTRESGVPDRFTGSVSGTDFTLTISSVQAEDLAVYYCQNDYNYPYTFGGG TKLEIKRLEHHHHHH | |
| H4P-GGGGS-SEQ ID NO 2 | MAQVKLQESGPELVRPGTSVKVSCKASGYAFTNYLI - HRRCNPNNKKR - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY WASTRESGVPDRFTGSVSGTDFTLTISSVQAEDLAVYYCQNDYNYPYTFGGG TKLEIKRLEHHHHHH | 57 |
| LMWP-GGGGS-SEQ ID NO 2 | MAQVKLQESGPELVRPGTSVKVSCKASGYAFTNYLI- VSRRRRRRGGRRRR - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY WASTRESGVPDRFTGSVSGTDFTLTISSVQAEDLAVYYCQNDYNYPYTFGGG TKLEIKRLEHHHHHH | 58 |
| hBD3-3-GGGGS-SEQ ID NO 2 | MAQVKLQESGPELVRPGTSVKVSCKASGYAFTNYLI - GKCSTRGRKCCRRKK - GGGGS - EWIKQRPGQGLEWIGVIHPGNGGTNYNENFKGKATLTADKSSSTAYMQLSSL TSDDSAVYFCASGNDGSYWGQGTTVTVSSGGGGSGKGGSGGGGSGGGGSDIE LTQSPSSLTVTAGEKVTMSCKSSQSLLNSGDQKIYLTWYQQKPGQPPKLLIY WASTRESGVPDRFTGSVSGTDFTLTISSVQAEDLAVYYCQNDYNYPYTFGGG TKLEIKRLEHHHHHHH | 59 |

Table 5 below shows the amino acid sequences of the fusion proteins wherein the cancer cell-penetrating peptide is linked to the KRAS mutant scFv and which are each cloned into the pcDNA3.4-TOPO for mammalian cell expression.

**[Table 5]**

| Fusion protein | Amino acid sequence | SEQ ID NO |
|---|---|---|
| SEQ ID NO 2-GGGGSGGGGSG GGGS -H4K | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD FTLTISSVQAEDLAVYYCQNDYNYPYTFGGGTK - GGGGSGGGGSGGGGS - HRRCNKNNKKR - LEIKRLEHHHHHH | 90 |
| SEQ ID NO 2-GGGGSGGGGSG GGGS -H4P | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD FTLTISSVQAEDLAVYYCQNDYNYPYTFGGGTK- GGGGSGGGGSGGGGS - HRRCNPNNKKR - LEIKRLEHHHHHH | 91 |
| SEQ ID NO 2-GGGGSGGGGSG GGGS -LMWP | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD | 92 |
| | FTLTISSVQAEDLAVYYCQNDYNYPYTFGGGTK - GGGGSGGGGSGGGGS - VSRRRRRRGGRRRR- LEIKRLEHHHHHH | |
| SEQ ID NO 2-GGGGSGGGGSG GGGS -hBD3-3 | MAQVKLQESGPELVRPGTSVKVSCKASGYAFTNYLIEWIKQRPGQGLEWIGV IHPGNGGTNYNENFKGKATLTADKSSSTAYMQLSSLTSDDSAVYFCASGNDG SYWGQGTTVTVSSGGGGSGGGGSGGGGSDIELTQSPSSLTVTAGEKVTMSCK SSQSLLNSGDQKIYLTWYQQKPGQPPKLLIYWASTRESGVPDRFTGSVSGTD FTLTISSVQAEDLAVYYCQNDYNYPYTFGGGTK - GGGGSGGGGSGGGGS - GKCSTRGRKCCRRKK - LEIKRLEHHHHHH | 93 |

pcDNA3.1-TOPO or pcDNA3.4-TOPO was digested with EcoR I and BamH I. The 900-960 bp KRAS mutant scFv gene fragment was recovered by an agarose electroelution method. To insert the gene fragment into pcDNA3.1-TOPO and pcDNA3.4-TOPO vectors, the gene was amplified by PCR, and at the same time, BamH I and EcoR I restriction enzyme sites were synthesized. Each digested vector and insert were mixed together in a ratio of 4 µl:4 µl, and ligated together using T4 DNA ligase in a ligation buffer containing 500 mM Tris-HCl, 100 mM MgCl₂, 200 mM DTT and 10 mM ATP at 4°C for 16 to 18 hours.

The present inventors transformed the prepared vector into the Chinese hamster ovary cell line Expi-CHO-S and the human embryonic kidney cell line Expi-293F. The Chinese hamster ovary cell line ExpiCHO-S was obtained from Thermo Fisher Scientific (USA) and cultured in ExpiCHO Expression medium (GIBCO, USA).

Next, the ExpiCHO-S cell line (3×10⁸ cells) was added to a 50 mL culture medium in a 250 mL disposable Erlenmeyer flask. 160 µL of ExpiFectamine CHO reagent (Gibco, Cat # A20130) diluted in 1.84 mL of Opti-MEM was added to 2 mL of Opti-MEM containing 50 µg of the plasmid and then incubated at room temperature for 5 minutes. Next, the dilution was evenly added dropwise to the prepared Chinese hamster ovary cells, and then incubated in a 8% CO₂ incubator at 37°C for 18 hours, and then 300 µL of ExpiCHO enhancer and 12 mL of ExpiCHO Feed were added thereto and incubated for 5 days.

The human embryonic kidney cell line Expi-293F was obtained from Thermo Fisher Scientific (USA) and cultured in Expi293 Expression medium (GIBCO, USA). Next, the Expi-293F cell line (1.50×10⁷ cells) was added to 50 mL of culture medium in a 250 mL disposable Erlenmeyer flask. 160 µL of ExpiFectamine 293 Reagent (Gibco, Cat # A14525) (Gibco, Cat # A14525) diluted in 2.8 mL of Opti-MEM was added to 3 mL of Opti-MEM containing 50 µg of the plasmid and then stationary incubated at room temperature for 20 minutes. Next, the dilution was evenly added dropwise to the prepared human embryonic kidney cells, and then incubated in an 8% CO₂ incubator at 37°C for 18 hours, and then 300 µL of ExpiFectamine 293 transfection enhancer 1 and 3 mL of ExpiFectamine 293 transfection enhancer 2 were added thereto and incubated for 3 days.

Six days after transduction, the culture medium was collected, sterilized and then purified using FPLC. After separation and purification was performed using a nickel chromatography column (HisTrap^{™} excel, GE Healthcare), the nickel column was equilibrated with a buffer [20mM sodium phosphate, 0.5M NaCl, pH7.4], and then medium was applied to the column. After washing using the above buffer containing 25 mM imidazole, the protein was separated by increasing the imidazole to 125 mM. The separated proteins were stored after removing the imidazole by desalting.

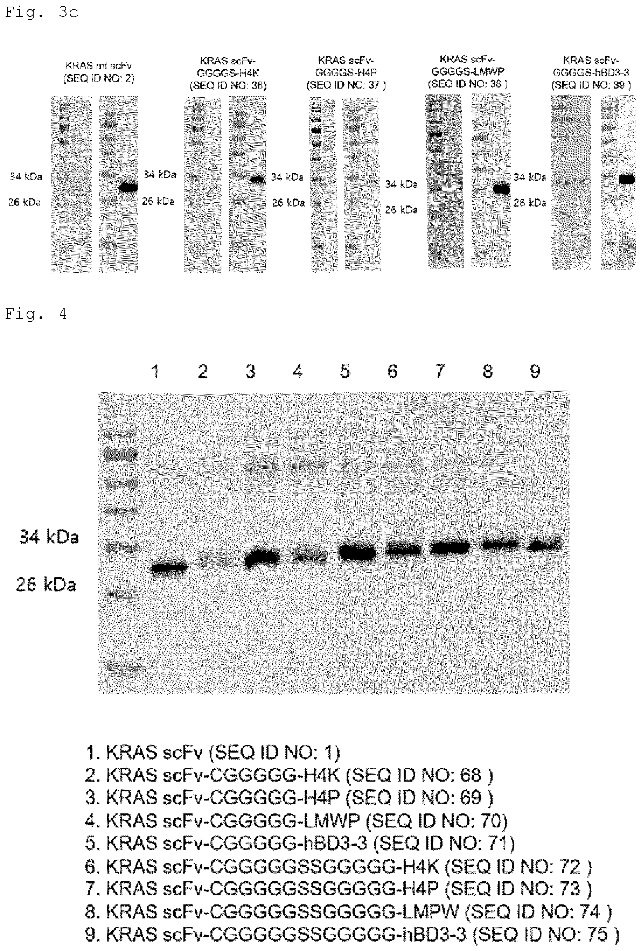

FIG. 3 shows the results of SDS-PAGE and Western blot analysis of the cancer cell-penetrating and KRAS mutant-targeting antibodies and scFvs expressed and purified in pcDNA3.4-TOPO (FIG. 3(B)) and pcDNA3.1-TOPO (FIG. 3(C)).

### Example 2: Production of Cancer Cell-Penetrating Peptide and Anti-KRAS Mutant Single-Chain Variable Fragment (scFv) by Chemical conjugation

### Example 2-1: Synthesis of Cell-Penetrating Peptide

Table 6 below shows the amino acid sequences of the cancer cell-penetrating peptide and liker used in the chemical conjugation method.

**[Table 6]**

| Linker type | Cancer cell-penetrating peptide (CCPP) | Sequence and SEQ ID NO of linker and cancer cell-penetrating peptide |
|---|---|---|
| CGGGGG | CCPP1 (H4K) | 60: CGGGGG-HRRCNKNNKKR |
| | CCPP2 (H4P) | 61: CGGGGG-HRRCNPNNKKR |
| | CCPP3 (LMWP) | 62: CGGGGG-VSRRRRRRGGRRRR |
| | CCPP4 (hBD3-3) | 63: CGGGGG-GKCSTRGRKCCRRKK |
| CGGGGGSSGGGGG | CCPP1 (H4K) | 64: CGGGGGSSGGGGG-RKKNNKNCRRH |
| | CCPP2 (H4P) | 65: CGGGGGSSGGGGG-RKKNNPNCRRH |
| | CCPP3 (LMWP) | 66: CGGGGGSSGGGGG-RRRRGGRRRRRRSV |
| | CCPP4 (hBD3-3) | 67: CGGGGGSSGGGGG-GKCSTRGRKCCRRKK |

Amino acids and reagents for synthesis were purchased from GL Biochem and Sigma-Aldrich. Peptides were synthesized from the C-terminus by an F-moc solid-state chemical synthesis method using a reaction vessel. That is, peptides were synthesized using Rink amide MBHA resin (0.678 mmol/g, 100 to 200 mesh) to which Fmoc-(9-fluorenylmethoxycarbonyl) as a blocking group was bound. After placing 1 g of Rink amide MBHA resin in the reaction vessel, the resin was swollen with DMF, and then 20% piperidine/DMF solution was used to remove the Fmoc-group. According to the sequence from the C-terminus, 0.5M amino acid solution (solvent: dimethylformamide, DMF), 1.0M DIPEA (solvent: dimethylformamide & n-methylpyrrolidone, DMF&NMP), 0.5M HBTU (solvent: dimethylformamide, DMF) were added in amounts of 5, 10 and 5 equivalents, respectively, and reacted for 1 to 2 hours under a nitrogen atmosphere. After each of the deprotection and coupling steps, washing three times with DMF and methanol was performed. Even after coupling the last amino acid, deprotection was performed to remove the Fmoc-group.

Confirmation of the synthesis was performed using the ninhydrin test method. The resin confirmed by the test to be completely synthesized was vacuum-dried, and a trifluoroacetic acid (TFA) cleavage cocktail was added thereto in an amount of 10 ml per g of the resin, followed by shaking for 3 hours. Then, the cocktail containing the peptide dissolved therein was separated from the resin by filtration. The peptide was crystallized into a solid state by placing the filtered solution in cold ether or adding an excess of cold ether directly to the TFA cocktail solution containing the peptide dissolved therein, and the peptide was collected by centrifugation. At this time, the TFA cocktail was completely removed through washing several times with ether and centrifugation. The peptide thus obtained was vacuum-dried.

The synthesized peptide was separated and purified by high-performance liquid chromatography (Shimadzu, Japan). Analysis was performed using a C₁₈ column (diameter: 4.6 mm) by running 0.1% TFA/H₂O and 0.1% TFA/acetonitrile at a flow rate of 1 ml/min with a gradient from 5 to 45%, and the wavelength of the UV detector was set to 220 nm. Purification was performed using a column (diameter: 50 mm) at a flow rate of 50 ml/min under the same solvent and detection wavelength conditions as above. The molecular weight of the purified peptide was analyzed through mass spectrometry.

### Example 2-2: Chemical conjugation of Cancer Cell-Penetrating Peptide to Linker Moiety of KRAS Mutant-Targeting scFv and Purification

1.02 mg of KRAS scFv of SEQ ID NO: 1 was dissolved in 1 mL of PBS buffer (pH 8.3). 2.12 mg of SPDP (succinimidyl 3-(2-pyridyldithio)propionate, ThermoFisher, 21857) was dissolved in 120 µL of DMSO (dimethyl sulfoxide). 40 µL of the SPDP solution was added to the KRAS scFv solution and allowed to react for 1 hour under a light-shielded condition, and this addition and reaction process was repeated three times. Alternatively, 2 mg sulfo-SMCC (succinimidyl 4-[N-maleimidomethyl]cyclohexane-1-carboxylate, ThermoFisher, 22322) was dissolved in PBS. 40 µL of the sulfo-SMCC solution was added to the KRAS scFv solution and allowed to react for 1 hour under a light-shielded condition, and this addition and reaction process was repeated three times.

After completion of the reaction, desalting was performed using a PD-10 desalting column (GE Healthcare), and 3.5 mL of pyridyldithiol activated KRAS scFv was obtained. To the pyridyldithiol activated KRAS scFv, a solution of each of 2.5 mg Cys-G5-H4K (SEQ ID NO: 60), Cys-G5-H4P (SEQ ID NO: 61), Cys-G5-LMWP (SEQ ID NO: 62), Cys-G5-hBD3-3 (SEQ ID NO: 63), Cys-G5S2G5-H4K (SEQ ID NO: 64), Cys-G5S2G5-H4P (SEQ ID NO: 65), Cys-G5S2G5-LMWP (SEQ ID NO: 66), and Cys-G5S2G5-hBD3-3 (SEQ ID NO: 67) in 300 µL of triple distilled water was added, and each mixture was adjusted to pH 8.3 using 1M tris buffer (pH=9). Each mixture was allowed to react at 4°C overnight under a light-shielded condition. The KRAS mutant scFv having each cancer cell-penetrating peptide bound thereto was purified by FPLC (Akta Pure, GE Healthcare) using a heparin column (HiTrap, GE Healthcare).

1.02 mg of scFv of SEQ ID NO: 1 was dissolved in 1 mL of PBS buffer (pH 7.4). 2.98 mg of Sulfo-SMCC (sulfosuccinimidyl 4-(N-maleimidomethyl)cyclohexane-1-carbocylate, ThermoFisher, catalog 22322) was dissolved in 300 µL of triple distilled water. 100 µL of the Sulfo-SMCC solution was added to the scFv solution and allowed to react for 30 minutes under a light-shielded condition, and this addition and reaction process was repeated three times. After completion of the reaction, desalting was performed using a PD-10 desalting column (GE Healthcare, 17-0851-01), and 3.5 mL of maleimide activated scFv was obtained. To the maleimide activated scFv, a solution of each of 2.5 mg Cys-G5-H4K (SEQ ID NO: 60), Cys-G5-H4P (SEQ ID NO: 61), Cys-G5-LMWP (SEQ ID NO: 62), Cys-G5-hBD3-3 (SEQ ID NO: 63), Cys-G5S2G5-H4K (SEQ ID NO: 64), Cys-G5S2G5-H4P (SEQ ID NO: 65), Cys-G5S2G5-LMWP (SEQ ID NO: 66), and Cys-G5S2G5-hBD3-3 (SEQ ID NO: 67) in 300 µL of PBS buffer (pH 8.3) was added, and each mixture was adjusted to pH 7.0 to 7.5. Each mixture was allowed to react at 4°C overnight under a light-shielded condition. The scFv having each cancer cell-penetrating peptide bound thereto was purified by FPLC (Akta Pure, GE healthcare) using a heparin column (HiTrap, GE Healthcare).

FIG. 4 shows the results of SDS-PAGE and Western blot analysis of the cancer cell-penetrating and KRAS mutant-targeting antibodies or single-chain variable fragments produced by the chemical conjugation method.

The molecular weight of the KRAS mutant scFv of SEQ ID NO: 1 was about 30 kDa, and the molecular weight of the KRAS mutant scFv having the cancer cell-penetrating peptide bound thereto was measured to be about 32 kDa. In addition, the molecular weight of the band bound to the KRAS antibody in the Western blot was measured to be 30 kDa for the scFv of SEQ ID NO: 1, and was measured to be about 32 kDa for the scFv having the cancer cell-penetrating peptide bound thereto, suggesting that the peptide was bound to the scFv.

### Example 2-3: Chemical Conjugation of Cancer Cell-Penetrating Peptide to C-Terminus of KRAS Mutant scFv and Purification

The KRAS mutant scFv of SEQ ID NO: 3 was allowed to react with 20 molar equivalents of tris(2-carboxyethyl)phosphine (TCEP) at room temperature for 3 hours to form a free thiol group. 100 molar equivalents of 5,50-dithiobis-(2-nitrobenzoic acid) (DTNB) was added to the KRAS mutant scFv solution and reacted for 1 hour at room temperature so as to bind to the free thiol group. Excess DTNB not bound to the KRAS mutant scFv was removed by FPLC (Akta Pure, GE healthcare) using a heparin column (HiTrap, GE Healthcare). Thereafter, the KRAS mutant scFv-DTNB conjugate was allowed to react with 10 molar equivalents of each cancer cell-penetrating peptide (SEQ ID NOs: 60 to 67) at room temperature for 1 hour. The scFv having each cancer cell-penetrating peptide bound thereto was purified by FPLC (Akta Pure, GE healthcare) using a heparin column (HiTrap, GE Healthcare).

Table 7 below shows the amino acid sequences of the fusion proteins wherein the cancer cell-penetrating peptide is linked to the KRAS mutant scFv and which were produced by the chemical conjugation method.

**[Table 7]**

| SEQ ID NO of conjugate comprising scFv of SEQ ID NO: 1 and cancer cell-penetrating peptide | SEQ ID NO of conjugate comprising scFv of SEQ ID NO: 1 and cancer cell-penetrating peptide |
|---|---|
| 68 : | 76: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K-**CGGGGG-HRRCNKNNKKR-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGG- HRRCNKNNKKR is linked to the amine group of the linker moiety of scFv. Lysine of the linker moiety in SEQ ID NO: 1 | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C**-CGGGGG-HRRCNKNNKKR-LEIKRGSENLYFQGGSGKPIPNPLLGLDSTGGSGG SHHHHHH CGGGGG- HRRCNKNNKKR is linked to the SH group of cysteine at the C-terminus. |
| 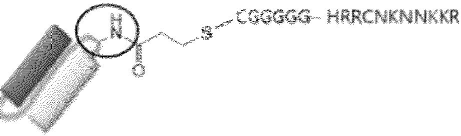 | 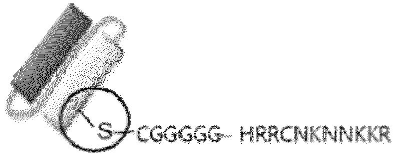 |
| | Cysteine at the C-terminus of SEQ ID NO: 3 |
| 69: | 77: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K**-CGGGGG-HRRCNPNNKKR-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGG- HRRCNPNNKKR is linked to the amine group of lysine in the linker moiety of scFv. Lysine of the linker moiety in SEQ ID NO: 1 | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C-**CGGGGG-HRRCNPNNKKR-LEIKRGSENLYFQGGSGKPIPNPLLGLDSTGGSGG SHHHHHH CGGGGG- HRRCNPNNKKR is linked to the SH group of cysteine at the C-terminus. |
| 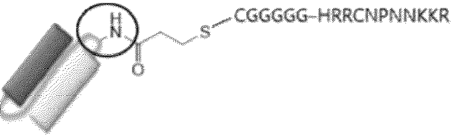 | 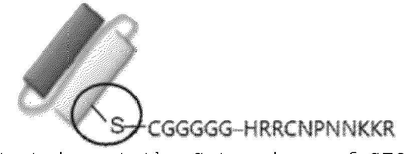 |
| | Cysteine at the C-terminus of SEQ ID NO: 3 |
| 70: | 78: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K-**CGGGGG-VSRRRRRRGGRRRR-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGG- VSRRRRRRGGRRRR is linked to the amine group of lysine in the linker moiety of scFv. Lysine of the linker moiety in SEQ ID NO: 1 | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C-**CGGGGG-VSRRRRRRGGRRRR-LEIKRGSENLYFQGGSGKPIPNPLLGLDSTGGSGG SHHHHHH CGGGGG- VSRRRRRRGGRRRR is linked to the SH group of cysteine at the C-terminus. |
| 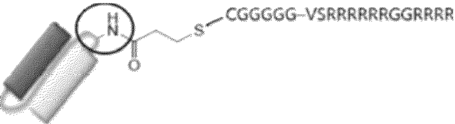 | 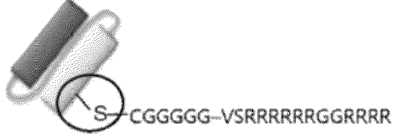 |
| | Cysteine at the C-terminus of SEQ ID NO: 3 |
| 71: | 79: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K**-CGGGGG-GKCSTRGRKCCRRKK-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGG- GKCSTRGRKCCRRKK is linked to the amine group of lysine in the linker moiety of scFv. Lysine of the linker moiety in SEQ ID NO: 1 | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTKC-CGGGGG-GKCSTRGRKCCRRKK-LEIKRGSENLYFQGGSGKPIPNPLLGLDSTGGSGG SHHHHHH CGGGGG- GKCSTRGRKCCRRKK is linked to the SH group of cysteine at the C-terminus. |
| 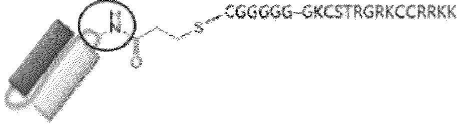 | 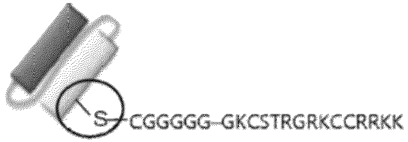 |
| | Cysteine at the C-terminus of SEQ ID NO: 3 |
| 72: | 80: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K-**CGGGGGSSGGGG-HRRCNKNNKKR-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGGSSGGGG- HRRCNKNNKKR is linked to the amine group of lysine in the linker moiety of scFv. | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C-**CGGGGGSSGGGG-HRRCNKNNKKR-LEIKRGSENLYFQGGSGKPIPNPLLGLDSTGGSGG SHHHHHH |
| Lysine of the linker moiety in SEQ ID NO: 1 | CGGGGGSSGGGG- HRRCNKNNKKR is linked to the SH group of cysteine at the C-terminus. |
| 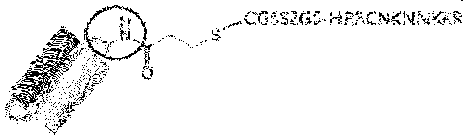 | 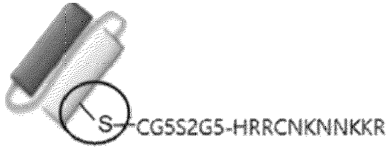 |
| | Cysteine at the C-terminus of SEQ ID NO: 3 |
| 73: | 81: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K**-CGGGGGSSGGGGG-RKKNNPNCRRH-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGGSSGGGGG- RKKNNPNCRRH is linked to the amine group of lysine in the linker moiety of scFv. Lysine of the linker moiety in SEQ ID NO: 1 | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C-**CGGGGGSSGGGGG-RKKNNPNCRRH-LEIKRGSENLYFQGGSGKPIPNPLLGLDSTGGSGG SHHHHHH CGGGGGSSGGGGG- RKKNNPNCRRH is linked to the SH group of cysteine at the C-terminus. |
| 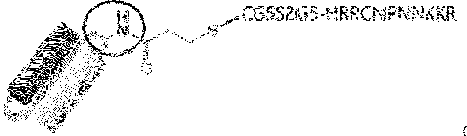 | 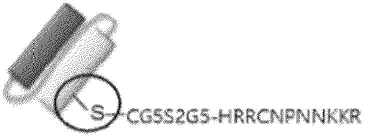 |
| | Cysteine at the C-terminus of SEQ ID NO: 3 |
| 74: | 82: |
| MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS G**K-**CGGGGGSSGGGGG-RRRRGGRRRRRRSV-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGGSSGGGGG-RRRRGGRRRRRRSV is linked to the amine group of lysine in the linker moiety of scFv. Lysine of the linker moiety in SEQ ID NO: 1 | MAWVWTLLFLMAAAQSIQAEWIKQRPGQGLEWIGV IHPGNGGTNYNENFKGKATLTADKSSSTAYMQLSS LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C-**CGGGGGSSGGGGG-VSRRRRGGRRRRRRSV-LEIKRGSENLYFQGGSGKPIPNPLLGLDSTGGSGG SHHHHHH CGGGGGSSGGGGG-VSRRRRGGRRRRRRSV is linked to the SH group of cysteine at the C-terminus. |
| 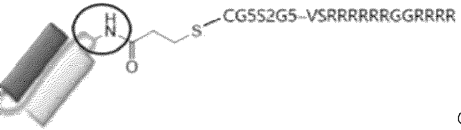 | 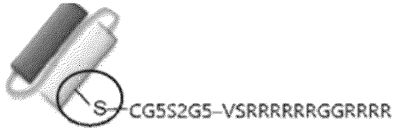 |
| | Cysteine at the C-terminus of SEQ ID NO: 3 |
| 75: | 83: |
| MAWVWTLLFLMAAAQSIQAEWIKQRPGQGLEWIGV IHPGNGGTNYNENFKGKATLTADKSSSTAYMQLSS | MAWVWTLLFLMAAAQSIQAEWIKQRPGQGLEWIGV IHPGNGGTNYNENFKGKATLTADKSSSTAYMQLSS |
| LTSDDSAVYFCASGNDGSYWGQGTTVTVSSGGGGS G**K-**CGGGGGSSGGGGG-GKCSTRGRKCCRRKK-GGSGGGGSGGGGSDIELTQSPSSLTVTAGEKVTMS CKSSQSLLNSGDQKIYLTWYQQKPGQPPKLLIYWA STRESGVPDRFTGSVSGTDFTLTISSVQAEDLAVY YCQNDYNYPYTFGGGTKLEIKRGSENLYFQGGSGK PIPNPLLGLDSTGGSGGSHHHHHH CGGGGGSSGGGGG- GKCSTRGRKCCRRKK is linked to the amine group of lysine in the linker moiety of scFv. Lysine of the linker moiety in SEQ ID NO: 1 | LTSDDSAVYFCASGNDGSYWGQGTTVTVSSGGGGS GKGGSGGGGSGGGGSDIELTQSPSSLTVTAGEKVT MSCKSSQSLLNSGDQKIYLTWYQQKPGQPPKLLIY WASTRESGVPDRFTGSVSGTDFTLTISSVQAEDLA VYYCQNDYNYPYTFGGGTK**C-**CGGGGGSSGGGGG-GKCSTRGRKCCRRKK-LEIKRGSENLYFQGGSGKPIPNPLLGLDSTGGSGG SHHHHHH CGGGGGSSGGGGG-GKCSTRGRKCCRRKK is linked to the SH group of cysteine at the C-terminus. |
| 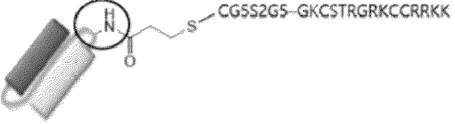 | 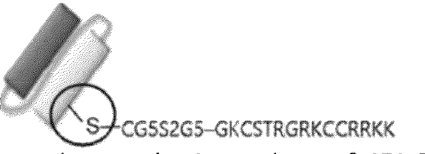 |
| | Cysteine at the C-terminus of SEQ ID NO: 3 |

### Example 3: Evaluation of Inhibitory Activity Against Cancer Cells with Different KRAS Mutant Expression Levels

In order to evaluate the cancer cell-specific growth inhibitory activities of the cancer cell-penetrating and KRAS mutant-targeting antibody and scFv according to the present invention, H358 cells (cancer cells expressing KRAS mutant) or human dermal fibroblasts (normal cells) were dispensed into a 96-well plate at a density of 5×10³ cells/well, and then cultured in RPMI medium 1640 (Gibco) for 24 hours. The cells were treated with each scFv (SEQ ID NOs: 1 and 28 to 35) at a concentration of 5 µM. Cytotoxicity was measured using CCK-8 (Cell Counting Kit-8, CK04, Dojindo Lab.). 48 hours after treatment with each scFv, the medium was removed, and 100 µl of CCK-8 solution was dispensed to fresh RPMI medium 1640. After 1 hour and 30 minutes, the absorbance at 450 nm was measured.

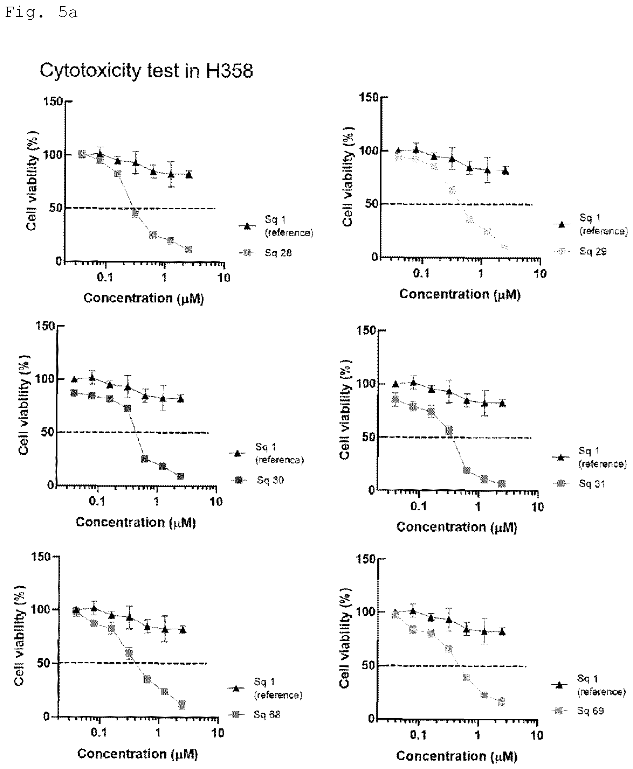

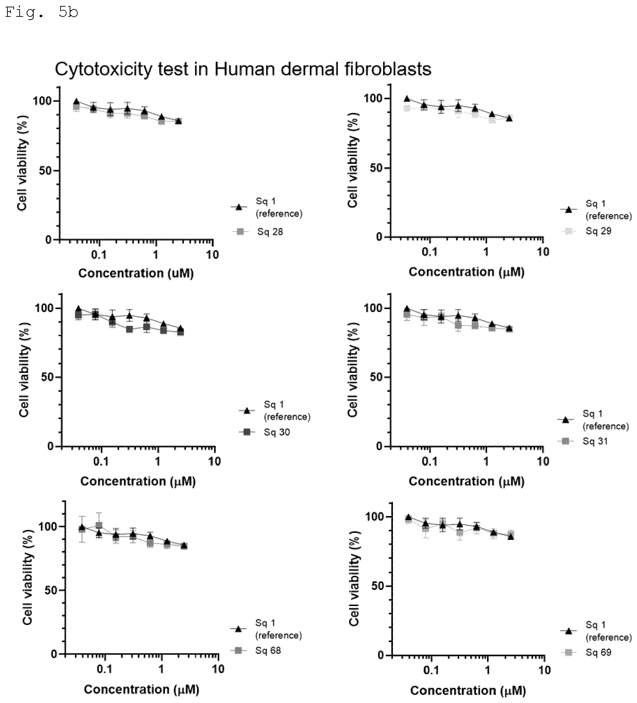

As a result, as shown in FIG. 5, it was confirmed that SEQ ID NOs: 28 to 31, 68 and 69 showed an IC₅₀ value of 100 to 500 nM (concentration at which 50% of cells are killed) in the H358 cells, and for SEQ ID NO: 1, the IC₅₀ value could not be measured even at a concentration of 5 µM. This is believed to be because SEQ ID NO: 1 did not show the cancer cell killing effect by being unable to penetrate the cancer cells, but SEQ ID NOs: 28 to 31, 68 and 69 exhibited cytotoxicity by being able to penetrate the cancer cells. For the human dermal fibroblasts that are normal cells, SEQ ID NOs: 28 to 31, 68 and 69 showed a cell viability of 80% or higher at all the concentrations. This means that the cancer cell-penetrating and KRAS mutant-targeting scFv conjugates have cancer cell-specific cytotoxicity.

### Example 4: Evaluation of active KRAS (Mutant KRAS) Expression Inhibitory Activities of Cancer Cell-Penetrating and KRAS Mutant-Targeting Antibody and scFv

1.0 × 10⁶ H358 cells (ATCC, CRL-5807) were seeded and cultured in RPMI medium 1640 (1X) (Gibco, 22400-089) for 24 hours, followed by starvation for 16 hours. The cells were treated with 50 ng/mL of EGF (epithelial growth factor, R&D Systems, 236-EG) for 10 minutes and treated with 1 µM of each variant. After 24 hours, the medium was removed, each well was washed with PBS, and the cells were harvested and centrifuged at 1,500 rpm for 3 minutes. 150 µL of 1x lysis buffer (1X Lysis/Binding/Wash buffer, 11524S) was added to and mixed with the cells and kept on ice for 5 minutes. The supernatant was separated by centrifugation at 16,000 rpm at 4°C for 15 minutes. Protein concentration was measured by BCA assay (Thermo Scientific, 23227). Washing was performed with 400 µL of 1x lysis buffer, and centrifugation was performed twice at 6000xg for 15 seconds.

To separate the mutant KRAS, 80 µg of GST-Raf1-RBD was added using Active GTPase Kit (Cell Signaling, 11860S), and 500 µg of the protein was dispensed into the spin cup. After 1 hour of incubation at 4°C, centrifugation was performed at 6000 xg for 15 seconds, and then the column was transferred into a fresh tube, and 400 µL of 1x lysis buffer was added thereto. After centrifugation at 6000 xg for 15 seconds, the column was transferred into a fresh tube, and 50 µL of 2x SDS buffer (containing 200 µl of 5X SDS sample loading dye and 300 µl of water) was added thereto and reacted for 2 minutes. After centrifugation at 6000 xg for 2 minutes, the resulting solution was held at 100°C for 7 minutes. The protein (20 µl protein loading volume/lane) was separated by 11% SDS-PAGE electrophoresis and transferred to a nitrocellulose membrane. To measure ERK1/2, which is a signal downstream of KRAS, 30 µg of the protein was electrophoresed by 11% SDS-PAGE and transferred to a nitrocellulose membrane. The membrane was blocked with 5% skim milk in T-TBS for 1 hour and incubated with a primary antibody (diluted 1:1000 in T-TBS) overnight with inversion at 4°C. The membrane was washed with T-TBS 3 times for 10 minutes per washing, and incubated with a secondary antibody (diluted 1:3000 in T-TBS) for 1 hour with inversion at room temperature. The membrane was washed 3 times with T-TBS for 10 minutes per washing, incubated with ECL chemiluminescence substrate (Thermo, 34580), and visualized with an Amersham Imager 680 (GE). Information on the antibodies used is shown in Table 8 below.

**[Table 8]**

| **No.** | **Antibody name** | **Manufacturer** | **Catalog No.** |
|---|---|---|---|
| **1** | GST | Abeam | Ab19256 |
| **2** | Active KRAS | Santa Cruz | SC-30 |
| **3** | RAS(E4K9L) | Cell Signaling | 91054S |
| **4** | p-B-Raf(S445) | Cell Signaling | 2696S |
| **5** | p-MEK1/2(S221) | Cell Signaling | 2338S |
| **6** | p-Erk1/2 | Santa Cruz | SC-7383 |
| **7** | GAPDH | Cell Signaling | 2118S |
| **8** | Goat anti-Rabbit IgG | BETHYL | A120-101P |
| **9** | Goat anti-Mouse IgG | BETHYL | A90-116P |

In SEQ ID NOs: 20 to 23, the linker between the KRAS mutant-targeting antibody or scFv and the cell-penetrating peptide is GGGGS, and in SEQ ID NOs: 90 to 93, the linker is GGGGSGGGGSGGGGS.

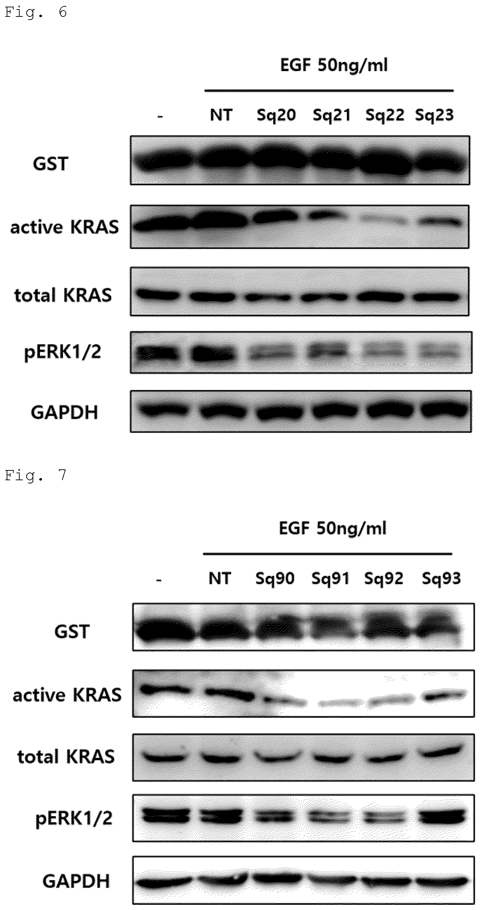

As a result, as shown in FIG. 6, it was confirmed that SEQ ID NOs: 20 to 23 all decreased active KRAS compared to treatment with EGF alone. In addition, it could be seen that pERK1/2, which is a signal downstream of KRAS, was also reduced by SEQ ID NOs: 20 to 23.

As shown in FIG. 7, it was confirmed that SEQ ID NOs: 90 to 93 all decreased active KRAS compared to treatment with EGF alone. In addition, it could be seen that pERK1/2, which is a signal downstream of KRAS, was also reduced by SEQ ID NOs: 91 to 92.

### Example 5: Evaluation of Cell Penetration Abilities of Cancer Cell-Penetrating and KRAS Mutant-Targeting Antibody and scFv

In order to test the cancer cell penetrating ability of the antibody or scFv, 1 × 10⁵ H358 cells were seeded, and after 48 hours, each of SEQ ID NOs: 20 to 23 was added thereto at a concentration of 500 nM, and the cells were cultured for 30 minutes. The cells were fixed with 4% paraformaldehyde, permeabilized with 0.1% Triton-X100 for 10 minutes, and blocked with 2% BSA. KRAS in the cells was reacted with an anti-RAS antibody (Origene, cst53270) at a dilution ratio of 1:1,000, and reacted with the secondary antibody anti-Rabbit Alexa Fluor 488 (Invitrogen, A27034) at a dilution ratio of 1:2,000. Each of SEQ ID NOs: 20 to 23 was reacted with a V5-Tag antibody (Origene, cst 13202) at a dilution ratio of 1:1,000, and reacted with anti-mouse Alexa Fluor 555 (Invitrogene, A28180) at a dilution ratio of 1:1,000. Cell nuclei were stained with 0.1 ug/mL DAPI (Thermofisher, R37606). SEQ ID NOs: 20 to 23 that penetrated the cells were measured with a confocal laser scanning microscope.

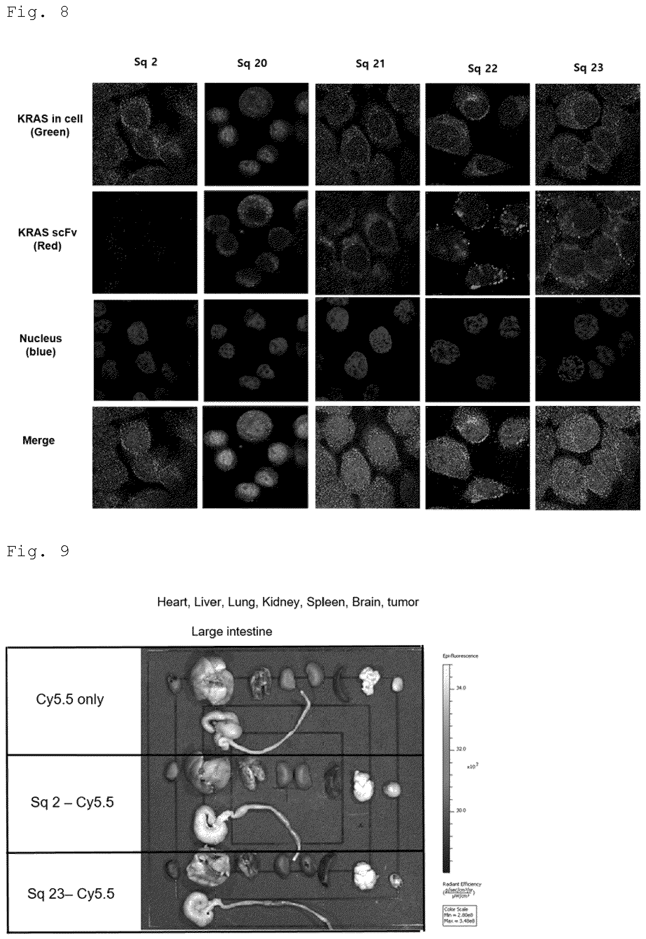

As a result, as shown in FIG. 8, it could be seen that all of SEQ ID NOs: 20 to 23, except for SEQ ID NO: 2, penetrated the cells. In addition, it could be seen that SEQ ID NOs: 20 to 23 co-localized with KRAS present in the cancer cells. Accordingly, it could be seen that SEQ ID NOs: 20 to 23 could penetrate the cells and bind to KRAS present in the cells.

### Example 6: Evaluation of Distribution of Cancer Cell-Penetrating and KRAS Mutant-Targeting Antibody and scFv in Cancer Tissue in Tumor Xenograft Animal Model

A mixture of 1 × 10⁶ H358 cells and 100 µl of Matrigel (BD Bioscience, San Diego, CA, USA) was transplanted into the thigh of each of 5-6 weeks old female Balb/c nude mice (5-6 weeks old; Japan SLC Inc., Hamamatsu, Japan) to form a tumor having a volume of 100 mm³. Each of Cy5.5-labeled SEQ ID NO: 2, Cy5.5-labeled SEQ ID NO: 23 and Cy5.5 was injected intraperitoneally at a dose of 20 ug per mouse, and after 24 hours, the distribution of fluorescence in each major organ and cancer tissue was observed.

As a result, as shown in FIG. 9, it could be seen that SEQ ID NO: 23 was distributed mainly in the cancer tissue 24 hours after injection, and a portion thereof was excreted by the kidney.

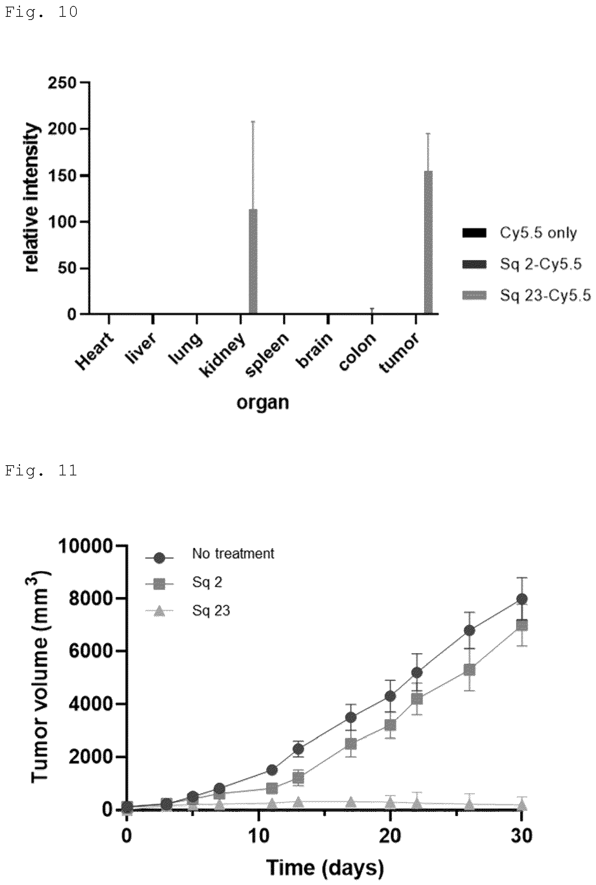

In addition, as shown in FIG. 10, it could be seen that, when the fluorescence intensity was measured, SEQ ID NO: 23 had the highest fluorescence intensity in the cancer tissue.

### Example 7: Evaluation of Tumor Inhibitory Activities of Cancer Cell-Penetrating and KRAS Mutant-Targeting Antibody and scFv in Tumor Xenograft Animal Model

A mixture of 1 × 10⁶ H358 cells and 100 µl of Matrigel (BD Bioscience, San Diego, CA, USA) was transplanted into the thigh of each of 5-6 weeks old female Balb/c nude mice (5-6 weeks old; Japan SLC Inc., Hamamatsu, Japan) to form a tumor having a volume of 100 mm³. Each of SEQ ID NO: 2 and SEQ ID NO: 23 was injected intraperitoneally at a dose of 1 mg/kg twice a week for 30 days. The volume of the tumor was measured with a vernier caliper at intervals of 3 to 4 days, and on day 30, the mice were euthanized and the tumor was excised and photographed.

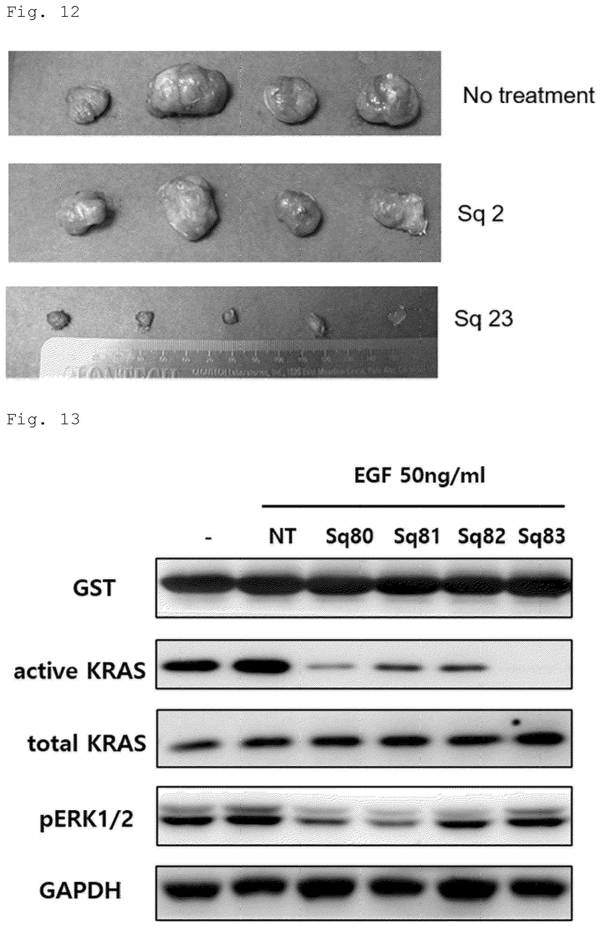

As a result, as shown in FIGS. 11 and 12, it was confirmed that SEQ ID NO: 2 did not have a tumor inhibitory effect, but SEQ ID NO: 23 had an excellent tumor inhibitory effect. This proves that SEQ ID NO: 23 that penetrated the cancer tissue effectively inhibits the growth of tumor cells.

### Example 8: Effects of Fusion Proteins of SEQ ID NOs: 80 to 83 Produced by Chemical Conjugation

### Example 8-1: Evaluation of Active KRAS (Mutant KRAS) Expression Inhibitory Activities of Cancer Cell-Penetrating and KRAS Mutant-Targeting Antibody and scFv

1.0 × 10⁶ H358 cells (ATCC, CRL-5807) were seeded and cultured in RPMI medium 1640 (1X) (Gibco, 22400-089) for 24 hours, followed by starvation for 16 hours. The cells were treated with 50 ng/mL of EGF (epithelial growth factor, R&D Systems, 236-EG) for 10 minutes and treated with 1 µM of each variant. The cells were cultured at 37°C for 24 hours. After 24 hours, the medium was removed, each well was washed with PBS, and the cells were harvested and centrifuged at 1,500 rpm for 3 minutes. 150 µL of 1x lysis buffer (1X Lysis/Binding/Wash buffer, 11524S) was added to and mixed with the cells and kept on ice for 5 minutes. The supernatant was separated by centrifugation at 16,000 rpm at 4°C for 15 minutes. Protein concentration was measured by BCA assay (Thermo Scientific, 23227). Washing was performed with 400 µL of 1x lysis buffer, and centrifugation was performed twice at 6000xg for 15 seconds.

To separate the mutant KRAS, 80 µg of GST-Raf1-RBD was added using Active GTPase Kit (Cell Signaling, 11860S), and 500 µg of the protein was dispensed into the spin cup. After 1 hour of incubation at 4°C, centrifugation was performed at 6000 xg for 15 seconds, and then the column was transferred into a fresh tube, and 400 µL of 1x lysis buffer was added thereto. After centrifugation at 6000 xg for 15 seconds, the column was transferred into a fresh tube, and 50 µL of 2x SDS buffer (containing 200 µl of 5X SDS sample loading dye and 300 µl of water) was added thereto and reacted for 2 minutes. After centrifugation at 6000 xg for 2 minutes, the resulting solution was heated at 100°C for 7 minutes. The protein (20 µl protein loading volume/lane) was separated by 11% SDS-PAGE electrophoresis and transferred to a nitrocellulose membrane. To measure ERK1/2 which is a signal downstream of KRAS, 30 µg of the protein was electrophoresed by 11% SDS-PAGE and transferred to a nitrocellulose membrane. The membrane was blocked with 5% skim milk in T-TBS for 1 hour and incubated with a primary antibody (diluted 1:1000 in T-TBS) overnight with inversion at 4°C. The membrane was washed with T-TBS 3 times for 10 minutes per washing, and incubated with a secondary antibody (diluted 1:3000 in T-TBS) for 1 hour while inversion at room temperature. The membrane was washed 3 times with T-TBS for 10 minutes per washing, incubated with ECL chemiluminescence substrate (Thermo, 34580), and visualized with Amersham Imager 680 (GE). Information on the antibodies used is shown in Table 9 below.

**[Table 9] Antibodies used**

| **No.** | **Antibody name** | **Manufacturers** | **Catalog No.** |
|---|---|---|---|
| **1** | GST | Abeam | Ab19256 |
| **2** | Active KRAS | Santa Cruz | SC-30 |
| **3** | RAS(E4K9L) | Cell Signaling | 91054S |
| **4** | p-B-Raf(S445) | Cell Signaling | 2696S |
| **5** | p-MEK1/2(S221) | Cell Signaling | 2338S |
| **6** | p-Erk1/2 | Santa Cruz | SC-7383 |
| **7** | GAPDH | Cell Signaling | 2118S |
| **8** | Goat anti-Rabbit IgG | BETHYL | A120-101P |
| **9** | Goat anti-Mouse IgG | BETHYL | A90-116P |

SEQ ID NOs: 80 to 83 were produced by linking the cell-penetrating peptide to the KRAS mutant-targeting antibody or scFv by chemical conjugation. As a result, as shown in FIG. 13, it was confirmed that SEQ ID NOs: 80 to 83 all decreased active KRAS compared to treatment with EGF alone. In addition, it could be seen that pERK1/2, which is a signal downstream of KRAS, was also decreased by SEQ ID NOs: 80 to 83.

### Example 8-2: Evaluation of Inhibitory Activities of Cancer Cell-Penetrating and KRAS Mutant-Targeting Antibody and scFv against Cancer Cells with Different KRAS Mutant Expression Levels

In order to evaluate the cancer cell-specific growth inhibitory activities of the cancer cell-penetrating and KRAS mutant-targeting antibody and scFv according to the present invention, H358 cells (cancer cells expressing KRAS mutant) or human dermal fibroblasts (normal cells) were dispensed into a 96-well plate at a density of 5×10³ cells/well, and then cultured in RPMI medium 1640 (Gibco) for 24 hours. The cells were treated with each of scFvs (SEQ ID NOs: 3 and 80 to 83) at a concentration of 0 to 5 µM. Cytotoxicity was measured using CCK-8 (Cell Counting Kit-8, CK04, Dojindo Lab.). Cytotoxicity was measured using CCK-8 (Cell Counting Kit-8, CK04, Dojindo Lab.). 48 hours after treatment with each scFv, the medium was removed, and 100 µl of CCK-8 solution was dispensed to fresh RPMI medium 1640. After 1 hour and 30 minutes, the absorbance at 450 nm was measured.

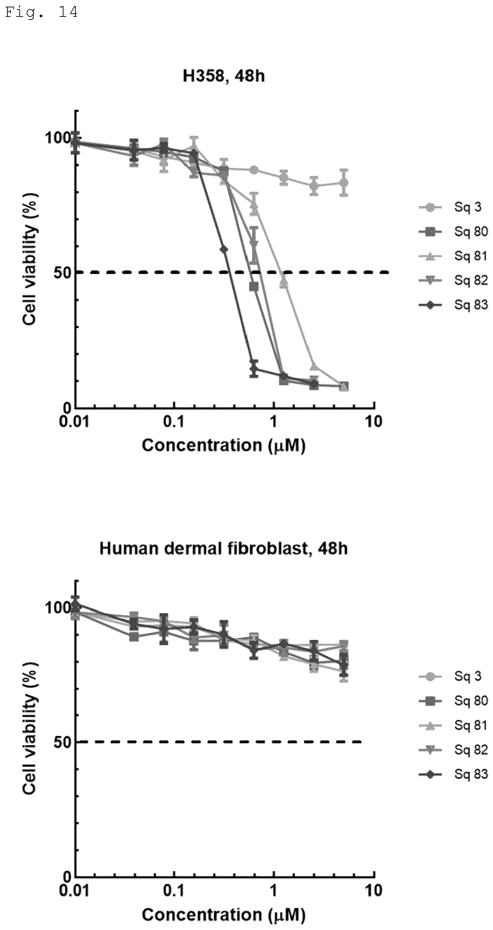

As a result, as shown in FIG. 14, it was confirmed that SEQ ID NOs: 80 to 83 showed an IC₅₀ value of 313 to 1250 nM (concentration at which 50% of cells are killed) in the H358 cells, and for SEQ ID NO: 3, the IC₅₀ value could not be measured even at a concentration of 5 µM. This is believed to be because SEQ ID NO: 3 did not show the cancer cell killing effect by being unable to penetrate the cancer cells, but SEQ ID NOs: 80 to 83 exhibited cytotoxicity by being able to penetrate the cancer cells. The human dermal fibroblasts that are normal cells showed a cell viability of 80% or higher at all the concentrations. This means that the cancer cell-penetrating and KRAS mutant-targeting scFv conjugates have cancer cell-specific cytotoxicity.

### Example 8-3: Evaluation of Cell Penetration Abilities of Cancer Cell-Penetrating and KRAS Mutant-Targeting Antibody and scFv

In order to test the cancer cell penetrating ability of the antibody or scFv, 1 × 10⁵ H358 cells were seeded, and after 48 hours, each of SEQ ID NOs: 80 to 83 was added thereto at a concentration of 500 nM, and the cells were cultured for 30 minutes. The cells were fixed with 4% paraformaldehyde, permeabilized with 0.1% Triton-X100 for 10 minutes, and blocked with 2% BSA. KRAS in the cells was reacted with an anti-RAS antibody (Origene, cst53270) at a dilution ratio of 1:1,000, and reacted with the secondary antibody anti-Rabbit Alexa Fluor 488 (Invitrogen, A27034) at a dilution ratio of 1:2,000. Each of SEQ ID NOs: 80 to 83 was reacted with a V5-Tag antibody (Origene, cst 13202) at a dilution ratio of 1:1,000, and reacted with an anti-mouse Alexa Fluor 555 (Invitrogene, A28180) at a dilution ratio of 1:1,000. Cell nuclei were stained with 0.1 ug/mL DAPI (Thermofisher, R37606). SEQ ID NOs: 80 to 83 that penetrated the cells were measured with a confocal laser scanning microscope.

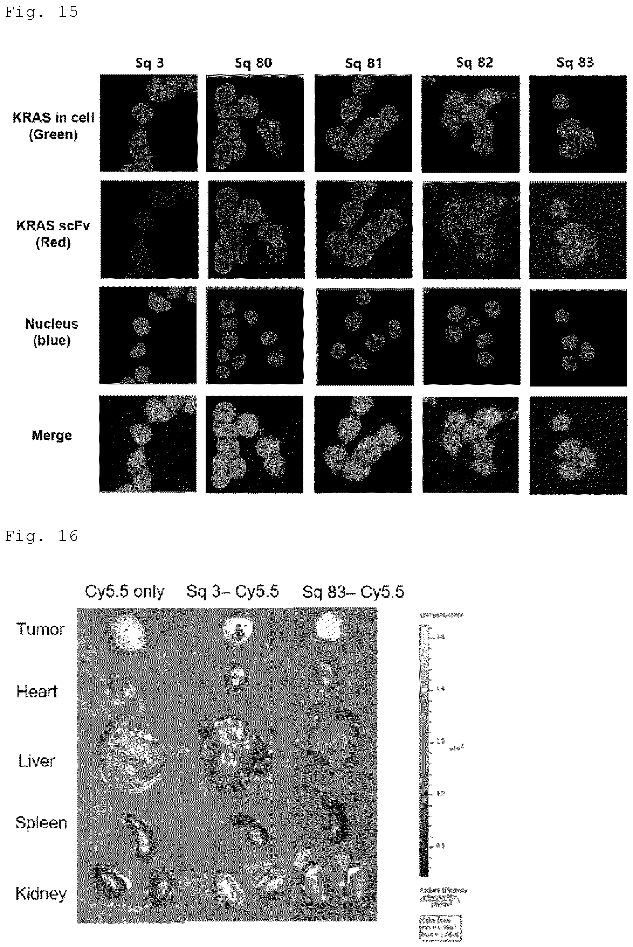

As a result, as shown in FIG. 15, it could be seen that all of SEQ ID NOs: 80 to 83, except for SEQ ID NO: 3, penetrated the cells. In addition, it could be seen that SEQ ID NOs: 80 to 83 co-localized with KRAS present in the cancer cells. Accordingly, it could be seen that SEQ ID NOs: 80 to 83 could penetrate the cells and bind to KRAS present in the cells.

### Example 8-4: Evaluation of Distribution of Cancer Cell-Penetrating and KRAS Mutant-Targeting Antibody and scFv in Cancer Tissue in Tumor Xenograft Animal Model

A mixture of 1 × 10⁶ H358 cells and 100 µl of Matrigel (BD Bioscience, San Diego, CA, USA) was transplanted into the thigh of each of 5-6 weeks old female Balb/c nude mice (5-6 weeks old; Japan SLC Inc., Hamamatsu, Japan) to form a tumor having a volume of 100 mm³. Each of Cy5.5-labeled SEQ ID NO: 3, Cy5.5-labeled SEQ ID NO: 83 and Cy5.5 was injected intraperitoneally at a dose of 20 ug per mouse, and after 24 hours, the distribution of fluorescence distributed in each major organ and cancer tissue was observed.

As a result, as shown in FIG. 16, it could be seen that SEQ ID NO: 83 was distributed mainly in the cancer tissue 24 hours after injection, and a portion thereof was excreted by the kidney.

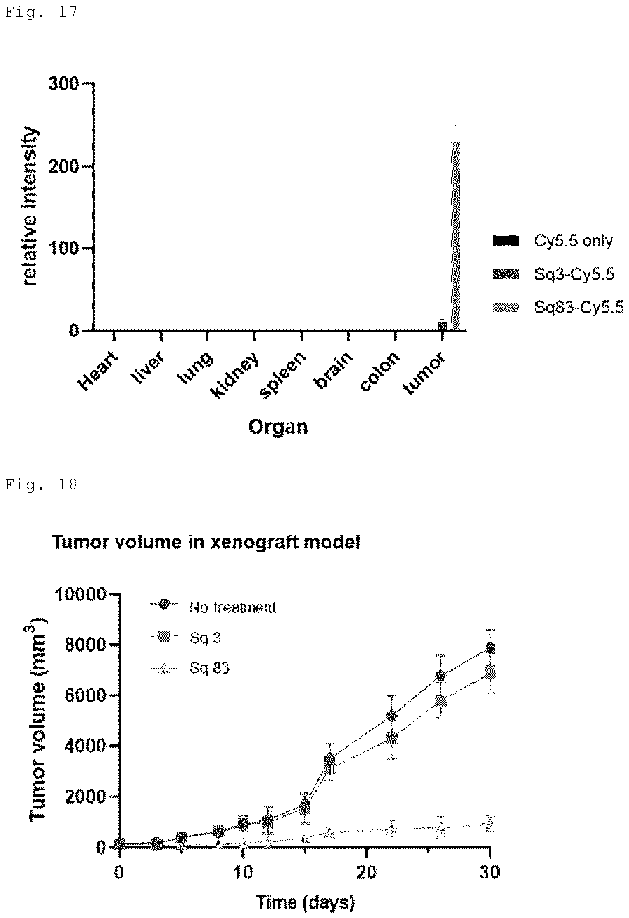

In addition, as shown in FIG. 17, it could be seen that, when the fluorescence intensity was measured, SEQ ID NO: 83 had the highest fluorescence intensity in the cancer tissue.

### Example 8-5: Evaluation of Tumor Inhibitory Activities of Cancer Cell-Penetrating and KRAS Mutant-Targeting Antibody and scFv in Tumor Xenograft Animal Model

A mixture of 1 × 10⁶ H358 cells and 100 µl of Matrigel (BD Bioscience, San Diego, CA, USA) was transplanted into the thigh of each of 5-6 weeks old female Balb/c nude mice (5-6 weeks old; Japan SLC Inc., Hamamatsu, Japan) to form a tumor having a volume of 100 mm³. Each of SEQ ID NO: 3 and SEQ ID NO: 83 was injected intraperitoneally at a dose of 1 mg/kg twice a week for 30 days. The volume of the tumor was measured with a vernier caliper at intervals of 3 to 4 days, and on day 30, the mice were euthanized and the tumor was excised and photographed.

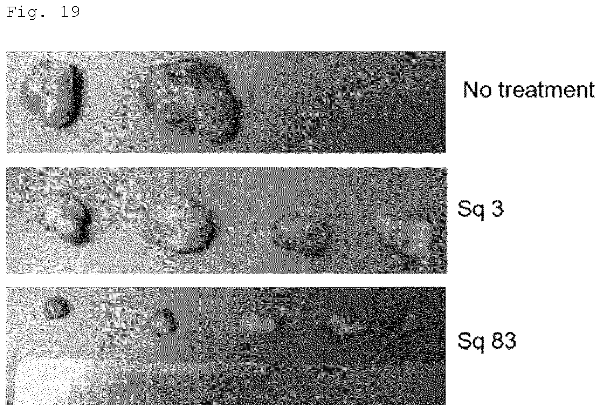

As a result, as shown in FIGS. 18 and 19, it was confirmed that SEQ ID NO: 3 did not have a tumor inhibitory effect, but SEQ ID NO: 83 had an excellent tumor inhibitory effect. This proves that SEQ ID NO: 83 that penetrated the cancer tissue effectively inhibits the growth of tumor cells.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

An intracellular oncogenic protein or a mutant thereof acts as an important factor in tumor pathogenesis. In the present invention, an intracellular oncogenic protein or oncogenic mutant protein-targeting antibody or a single-chain variable fragment thereof is used which inhibits the function of the intracellular oncogenic protein or mutant thereof by binding thereto. To enhance the accessibility of the antibody or single-chain variable fragment thereof to cancer cells, a cancer cell-penetrating peptide is linked to the antibody or single-chain variable fragment thereof. The fusion protein produced as described above may effectively penetrate tumor cells, thereby maximizing the antitumor or anticancer effect of the antibody or single-chain variable fragment thereof that targets the intracellular oncogenic protein or oncogenic mutant protein.

### Sequence List Free Text

An electronic file is attached.

## Claims

1. A fusion protein in which (i) an antibody or a single-chain variable fragment thereof, which targets an intracellular oncogenic protein or oncogenic mutant protein, is linked with (ii) a cancer cell-penetrating peptide.

2. The fusion protein of claim 1, wherein the intracellular oncogenic protein or oncogenic mutant protein is KRAS or androgen receptor.

3. The fusion protein of claim 1, wherein the antibody or single-chain variable fragment thereof is selected from the group consisting of SEQ ID NOs: 1 to 3.

4. The fusion protein of claim 1, wherein the cancer cell-penetrating peptide is selected from the group consisting of SEQ ID NO: 86 to SEQ ID NO: 89.

5. The fusion protein of any one of claims 1 to 4, wherein the cancer cell-penetrating peptide is linked via a linker to the N-terminus or C-terminus of the antibody or single-chain variable peptide thereof.

6. The fusion protein of claim 5, wherein the linker is GGGGS or GGGGSGGGGSGGGGS.

7. The fusion protein of any one of claims 1 to 4, which is a fusion protein wherein any one cancer cell-penetrating peptide selected from the group consisting of SEQ ID NO: 86 to SEQ ID NO: 89 is linked via a linker to a lysine or cysteine residue of any one single-chain variable fragment selected from the group consisting of SEQ ID NOs: 1 to 3.

8. The fusion protein of claim 7, wherein the linker is CGGGGG or CGGGGGSSGGGGG.

9. The fusion protein of claim 1, which is represented by any one of the amino acid sequences of SEQ ID NO: 12 to SEQ ID NO: 59, SEQ ID NO: 68 to SEQ ID NO: 83, and SEQ ID NO: 90 to SEQ ID NO: 93.

10. A nucleic acid encoding the fusion protein of claim 1.

11. A recombinant vector into which the nucleic acid of claim 10 has been introduced.

12. A recombinant cell into which the recombinant vector of claim 11 has been introduced.

13. The recombinant cell of claim 12, wherein the recombinant cell is an *E. coli* or mammalian cell.

14. A method for producing the fusion protein of claim 1 comprising steps of:
(a) expressing the fusion protein of claim 1 by culturing the recombinant vector of claim 12; and
(b) recovering the expressed fusion protein.

15. A pharmaceutical composition for treating a tumor comprising the fusion protein of any one of claims 1 to 9 as an active ingredient.

16. The pharmaceutical composition of claim 15, wherein the tumor is at least one selected from the group consisting of non-small cell lung cancer, colorectal cancer, pancreatic cancer, bladder cancer, kidney cancer, thyroid cancer, breast cancer, colon cancer, liver cancer, brain tumor, skin cancer, melanoma, colorectal cancer, prostate cancer, and blood cancer.
